# EUROPÄISCHE PATENTSCHRIFT

(11) **EP 2 131 830 B1**
(45) Veröffentlichungstag und Bekanntmachung des Hinweises auf die Patenterteilung: **10.05.2017**
(21) Anmeldenummer: 08716192.3
(22) Anmeldetag: 03.03.2008
(51) Int. Cl.: A61K 31/137, A61K 9/16, A61K 9/48

(54) **DARREICHUNGSFORM MIT ERSCHWERTEM MISSBRAUCH**
PHARMACEUTICAL FORM WITH IMPEDED ABUSE
FORME PHARMACEUTIQUE RENDANT L'USAGE ABUSIF PLUS DIFFICILE

(30) Priorität: 07.03.2007 DE 102007011485
(43) Veröffentlichungstag der Anmeldung: 16.12.2009
(73) Patentinhaber: Grünenthal GmbH, 52078 Aachen (DE)
(72) Erfinder: ARKENAU-MARIC, Elisabeth, 50931 Köln (DE); BARTHOLOMÄUS, Johannes, 52080 Aachen (DE); ZIEGLER, Iris, 86356 Neussäss (DE); HAUPTS, Marcel, 52224 Stolberg (DE)
(74) Vertreter: Bülle, Jan
(86) Internationale Anmeldenummer: PCT/EP2008/001672
(87) Internationale Veröffentlichungsnummer: WO 2008/107149

(56) Entgegenhaltungen:
- EP-A- 1 138 321
- DE-A1- 10 336 400
- DE-A1-102004 032 051
- DE-A1-102005 005 446

## Beschreibung

Die vorliegende Offenbarung betrifft eine multipartikuläre Darreichungsform mit erschwertem Missbrauch enthaltend neben einem oder mehreren Wirkstoffen mit Missbrauchspotential (A) gegebenenfalls wenigstens einen physiologisch verträglichen Hilfsstoff (B), mindestens ein synthetisches oder natürliches Polymeres (C), gegebenenfalls mindestens ein Wachs (D) und wenigstens ein Sprengmittel (E), wobei die einzelnen Partikel der Darreichungsform eine Bruchfestigkeit von mindestens 500 N und eine Wirkstofffreisetzung von wenigstens 75% nach 45 Minuten aufweisen sowie Verfahren zur Herstellung der offenbarten Darreichungsform.

Eine Vielzahl von pharmazeutischen Wirkstoffen weist neben einer ausgezeichneten Wirksamkeit auf ihrem betreffenden Anwendungsgebiet auch ein Missbrauchspotential auf, d.h. sie können von einem Missbraucher eingesetzt werden, um Wirkungen herbeizuführen, die nicht ihrem Bestimmungszweck entsprechen.

So werden beispielsweise Opiate, die eine exzellente Wirksamkeit bei der Bekämpfung von starken bis sehr starken Schmerzen zeigen, von Missbrauchern häufig zum Einleiten rauschartiger, euphorisierender Zustände verwendet. Insbesondere Wirkstoffe mit psychotroper Wirkung werden dementsprechend missbraucht.

Um Missbrauch zu ermöglichen, werden die entsprechenden Darreichungsformen wie Tabletten oder Kapseln vom Missbraucher zerkleinert, z. B. gemörsert, der Wirkstoff aus dem so erhaltenen Pulver mit Hilfe einer vorzugsweise wässrigen Flüssigkeit extrahiert und die resultierende Lösung, ggf. nach Filtration durch Watte oder Zellstoff, parenteral, insbesondere intravenös, appliziert. Bei dieser Art der Verabreichung kommt es zu einem gegenüber der oralen, missbräuchlichen Applikation noch zusätzlich beschleunigten Anfluten des Wirkstoffes mit dem vom Missbraucher gewünschten Ergebnis, nämlich dem Kick. Dieser Kick bzw. diese rauschartigen, euphorischen Zustände werden auch erreicht, wenn die gepulverte Darreichungsform nasal appliziert, d. h. geschnupft wird.

Zur Verhinderung dieser Missbrauchsmöglichkeiten wurde in dem US-A4,070,494 vorgeschlagen, der Darreichungsform ein quellbares Mittel zuzusetzen. Dieses quillt bei der Zugabe von Wasser zur Extraktion des Wirkstoffes auf und bewirkt, dass das vom Gel separierte Filtrat nur eine sehr geringe Wirkstoffmenge enthält.

Ein entsprechender Ansatz zur Verhinderung von parenteralem Missbrauch liegt auch der in der WO 95/20947 offenbarten Mehrschichttablette zugrunde, die den Wirkstoff mit Missbrauchspotential und mindestens einen Gelbildner jeweils in unterschiedlichen Schichten getrennt aufweist.

Ein weiterer Ansatz zur Verhinderung des parenteralen Missbrauchs wird in der WO 03/015531 A2 offenbart. Dort wird eine Darreichungsform enthaltend ein analgetisches Opioid und einen Farbstoff als aversives Mittel beschrieben. Die Farbe, die durch unzulässige Manipulation der Darreichungsform freigesetzt wird, soll den Missbraucher davon abhalten, diese manipulierte Darreichungsform zu verwenden.

Eine weitere bekannte Möglichkeit zur Erschwerung des Missbrauchs besteht darin, der Darreichungsform Antagonisten der Wirkstoffe, wie z. B. Naloxon oder Naltrexon im Fall von Opioiden, oder Verbindungen, die zu physiologischen Abwehrreaktionen führen, wie z. B. Radix Ipecacuanha = Brechwurz, der Darreichungsform zuzusetzen.

Es ist auch schon bekannt, den Missbrauch zu verhindern, indem die für einen Missbrauch notwendige Pulverisierung der Darreichungsformen mit den einem potentiellen Missbraucher üblicherweise zur Verfügung stehenden Mitteln erschwert bzw. verhindert wird. Entsprechend feste Darreichungsformen enthaltend Wirkstoffe mit Missbrauchspotential, die bei bestimmungsgemässer Applikation die gewünschte therapeutische Wirkung gewährleisten, aus deren aber die Wirkstoffe nicht durch einfaches Pulverisieren in eine zum Missbrauch geeignete Form übergeführt werden können, sind aus DE-A-103 36 400.5 bekannt.

DE 10 2005 005 446 offenbart eine Darreichungsform umfassend eine physiologisch wirksame Substanz mit zumindest teilweise retardierter Freisetzung; gegebenenfalls einen oder mehrere physiologisch verträgliche Hilfsstoffe; und ein synthetisches oder natürliches Polymer; wobei die Darreichungsform eine Bruchfestigkeit von mindestens 400 N aufweist.

In D 103 36 400 wird eine gegen Missbrauch gesicherte, thermogeforme Darreichungsform und Verfahren zu deren Herstellung beschrieben, die neben einem oder mehreren Wirkstoffen mit Missbrauchspotential sowie ggf. physiologisch verträglichen Hilfsstoffen mindestens ein synthetisches oder natürliches Polymer mit einer Bruchfestigkeit von mindestens 500 N enthält.

EP 1 138321 betrifft orale Darreichungsformen mit retardierter Wirkstofffreisetzung und hoher mechanischer Stabilität enthaltend einen oder mehrere Wirkstoffe, eine formulierte Mischung aus Polyvinylacetat und Polyvniylpyrrolidon, wasserlösliche Polymere oder nieder- oder hochmolekulare lipophile Zusatzstoffe, sowie weitere, übliche Hilfsstoffe, sowie deren Verwendung und Herstellung.

Druckschrift DE 10 2004 032 051 offenbart ein Verfahren zur Herstellung einer gegen Missbrauch gesicherten, festen Darreichungsform, enthaltend wenigstens einen Wirkstoff mit Missbrauchspotential und ein synthetisches oder natürliches Polymeres mit einer Bruchfestigkeit von ≥ 500 N, dadurch gekennzeichnet, dass eine entsprechende Mischung mithilfe eines Planetwalzen-Extruders durch eine Schmelzextrusion verarbeitet wird.

Diese gegen Missbrauch gesicherten Darreichungsformen zeichnen sich durch eine kontrollierte, vorzugsweise retardierte, Freisetzung des Wirkstoffs mit Missbrauchspotential aus. Für zahlreiche therapeutische Anwendungen, wie z. B. die Schmerzbekämpfung mit Hilfe von Wirkstoffen mit Missbrauchspotential, ist eine schnelle Freisetzung des Wirkstoffs notwendig.

Es war daher Aufgabe der vorliegenden Erfindung, eine Darreichungsform enthaltend einen Wirkstoff mit Missbrauchspotential zur Verfügung zu stellen, deren Missbrauch zumindest erschwert ist und die eine reproduzierbare, schnelle Freisetzung des Wirkstoffs mit Missbrauchspotential gewährleistet.

Diese Aufgabe wird durch die Bereitstellung der erfindungsgemässen, multipartikulären Darreichungsform mit erschwertem Missbrauchspotential umfassend
- wenigstens einen Wirkstoff mit Missbrauchspotential (A), welcher eine psychotrope Wirkung aufweist, ausgewählt aus der Gruppe der Opioide;
- wenigstens ein synthetisches oder natürliches Polymeres (C);
- gegebenenfalls wenigstens ein natürliches, halbsynthetisches oder synthetisches Wachs (D);
- wenigstens ein Sprengmittel (E), welches zumindest teilweise mit den Partikeln der Darreichungsform vermischt ist;
- einen Zusatzhilfsstoff (B2), der nicht Bestandteil der Partikel ist, ausgewählt aus der Gruppe der Füllstoffe;
- gegebenenfalls einen oder mehrere, weitere physiologisch verträgliche Hilfsstoffe (B),
wobei die einzelnen Partikel der Darreichungsform eine Bruchfestigkeit von mindestens 500 N und eine Wirkstoff-Freisetzung von wenigstens 75% nach 45 Minuten gemessen nach Pharm. Eur. in der Blattrührapparatur mit Sinker in 600 ml wässriger Pufferlösung mit einem pH-Wert von 1 ,2 bei 37 °C und 75 Umdrehungen pro min aufweisen,
gelöst.

Durch den Einsatz von Polymeren mit einer Mindestbruchfestigkeit von mindestens 500 N (gemessen, wie in der Anmeldung angegeben) in solchen Mengen, dass auch die Partikel der erfindungsgemässen Darreichungsform eine solche Mindestbruchfestigkeit von mindestens 500 N aufweisen, gelingt es, ein Pulverisieren der Darreichungsform mit üblichen Mitteln zu verhindern und damit den anschliessenden Missbrauch erheblich zu erschweren bzw. zu unterbinden.

Ohne ausreichende Zerkleinerung ist nämlich eine parenterale, insbesondere intravenöse, gefahrlose Applikation oder eine missbräuchliche nasale Anwendung nicht möglich, so dass die dadurch erzielten rauschartigen, euphorischen Zustände in der gewünschten Intensität und Schnelligkeit nicht erreichbar sind.

Unter einer Zerkleinerung wird erfindungsgemäss die Pulverisierung der Darreichungsform mit üblichen Mitteln, die einem Missbraucher üblicherweise zur Verfügung stehen, wie z. B. ein Mörser und Pistill, ein Hammer, ein Schlegel oder andere gebräuchliche Mittel zum Pulverisieren unter Krafteinwirkung verstanden.

Die erfindungsgemässen, multipartikulären Darreichungsformen sind daher zur Verhinderung des parenteralen und/oder nasalen Missbrauchs von Wirkstoffen, vorzugsweise von pharmazeutischen Wirkstoffen, mit Missbrauchspotential geeignet und gewährleisten aufgrund ihrer erfindungsgemässen Zusammensetzung eine rasche, kontrollierte Freisetzung des Wirkstoffs. Sie entsprechen daher den sogenannten IR-Darreichungsformen (immediate release-Darreichungsformen), da das Freisetzungsprofil des Wirkstoffs den entsprechenden Standardbedingungen genügt. Ganz besonders bevorzugt zeigen die erfindungsgemässen, multipartikulären Darreichungsformen eine Freisetzung des Wirkstoffs innerhalb von 1 bis 30 Minuten.

Pharmazeutische Wirkstoffe mit Missbrauchspotential sind dem Fachmann ebenso wie deren einzusetzende Mengen und Verfahren zu deren Herstellung bekannt und können als solche, in Form ihrer dementsprechenden Derivate, insbesondere Ester oder Ether, oder jeweils in Form entsprechender physiologisch verträglicher Verbindungen, insbesondere in Form ihrer entsprechenden Salze oder Solvate, als Racemate oder Stereoisomere in der erfindungsgemässen Darreichungsform vorliegen. Die erfindungsgemässe, multipartikuläre Darreichungsform eignet sich auch für die Verabreichung von mehreren pharmazeutischen Wirkstoffen in einer Darreichungsform. Vorzugsweise erhält die Darreichungsform nur einen bestimmten Wirkstoff mit Missbrauchspotential.

Die erfindungsgemässe, schnell freisetzende Darreichungsform eignet sich zur Erschwerung bzw. Verhinderung des Missbrauchs wenigstens eines pharmazeutischen Wirkstoffs mit Missbrauchspotential, welcher eine psychotrope Wirkung aufweist, der ausgewählt ist aus der Gruppe umfassend Opioide, vorzugsweise aus der Gruppe umfassend Tranquillantien, Benzodiazepine, Barbiturate, Stimulantien und weiteren Betäubungsmitteln.

Ganz besonders eignet sich die erfindungsgemässe Darreichungsform zur Erschwerung bzw. Verhinderung des Missbrauchs eines Opioids, Tranquillanz oder eines anderen Betäubungsmittels, das ausgewählt ist aus der Gruppe umfassend N{1-[2-(4-Ethyl-5-oxo-2-tetrazolin-1-yl)ethyl]-4-methoxymethyl-4-piperidyljpropionanilid (Alfentanil), 5,5-Diallylbarbitursäure (Allobarbital), Allylprodin, Alphaprodin, 8-Chlor-1-methyl-6-phenyl-4*H*[1,2,4]triazolo[4,3-a][1,4]-benzodiazepin (Alprazolam), 2-Diethylaminopropiophenon (Amfepramon), (±)-[α]-Methylphenethylamin (Amfetamin), 2-(α-Methylphenethylamino)-2-phenylacetonitril (Amfetaminil), 5-Ethyl-5-isopentylbarbitursäure (Amobarbital), Anileridin, Apocodein, 5,5-Diethylbarbitursäure (Barbital), Benzylmorphin, Bezitramid, 7-Brom-5-(2-pyridyl)-1 H-1,4-benzodiazepin-2(3*H*)-on (Bromazepam), 2-Brom-4-(2-chlorphenyl)-9-methyl-6H-thieno[3,2-f][1,2,4]triazolo[4,3-a][1,4]diazepin (Brotizolam), 17-Cyclopropylmethyl-4,5α-epopxy-7α[(S)-1-hydroxy-1,2,2-trimethyl-propyl]-6-methoxy-6,14-*endo*-ethanomorphinan-3-ol (Buprenorphin), 5-Butyl-5-ethylbarbitursäure (Butobarbital), Butorphanol, (7-Chlor-1,3-dihydro-1-methyl-2-oxo-5-phenyl-2H-1,4-benzodiazepin-3-yl)-dimethyl-carbamat (Camazepam), (1*S*,2*S*)-2-Amino-1-phenyl-1-propanol (Cathin/D-Norpseudoephedrin), 7-Chlor-*N-*methyl-5-phenyl-3*H*-1 ,4-benzodiazepin-2-ylamin4-oxid (Chlordiazepoxid), 7-Clor-1-methyl-5-phenyl-1*H*-1,5-benzodiazepin2,4(3*H*,5*H*)-dion (Clobazam), 5-(2-Chlorphenyl)-7-nitro-1*H*-1,4-benzodiazepin-2(3*H*)- on (Clonazepam), Clonitazen, 7-Chlor-2,3-dihydro-2-oxo-5-phenyl-1*H-*1,4-benzodiazepin-3-carbonsäure (Clorazepat), 5-(2-Chlorphenyl)-7-ethyl-1-methyl-1*H-*thieno[2,3-e][1,4]diazepin-2(3H)-on (Clotiazepam), 10-Chlor-11 b-(2chlorphenyl)- 2,3,7,11b-tetrahydrooxazolo[3,2-*d*][1,4]benzodiazepin-6(5*H*)-on (Cloxazolam), (-)-Methyl-[3β-benzoyloxy-2β(1α*H*,5α*H*)-tropancarboxylat] (Cocain), 4,5α-Epoxy-3-methoxy-17-methyl-7-morphinen-6α-ol (Codein), 5-(1-Cyclohexenyl)-5-ethylbarbitursäure (Cyclobarbital), Cyclorphan, Cyprenorphin, 7-Chlor-5-(2-chlorphenyl)-1*H*-1,4-benzodiazepin-2(3*H*)-on (Delorazepam), Desomorphin, Dextromoramid, (+)-(1-Benzyl-3-dimethylamino-2-methyl-1-phenylpropyl)propionat (Dextropropoxyphen), Dezocin, Diampromid, Diamorphon, Diamorphin (Heroin), 7-Chlor-1-methyl-5-phenyl-1*H*-1,4-benzodiatepin-2(3*H*)-on(Diazepam), 4,5α-Epoxy-3-methoxy-17-methyl-6α-morphinanol (Dihydrocodein), 4,5α-Epoxy-17-methyl-3,6a-morphinandiol (Dihydromorphin), Dimenoxadol, Dimephetamol, Dimethylthiambuten, Dioxaphetylbutyrat, Dipipanon, (6a*R*,10a*R*)-6,6,9-Trimethyl-3-pentyl-6a,7,8,10a-tetrahydro-6*H*-benzo[c]chromen-1-ol (Dronabinol), Eptazocin, 8-Chlor-6-phenyl-4*H-*[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1*H*-1,4 benzodiazepin-3-carboxylat] (Ethylloflazepat), 4,5α-Epoxy-3-ethoxy-17-methyl-7-morphinen-6α-ol (Ethylmorphin), Etonitazen, 4,5α-Epoxy-7α-(1-hydroxy-1-methylbutyl)-6-methoxy-17-methyl-6,14-*endo*-etheno-morphinan-3-ol (Etorphin), *N-*Ethyl-3-phenyl-8,9,10-trinorbornan-2-ylamin (Fencamfamin), 7-[2-(α-Methylphenethylamino)ethyl]-theophyllin) (Fenetyllin), 3-(α-Methylphenethylamino)propionitril (Fenproporex), *N*-(1-Phenethyl-4-piperidyl)propionanilid (Fentanyl), 7-Chlor-5-(2-fluorphenyl)-1-methyl-1*H*-1,4-benzodiazepin-2(3*H*)-on (Fludiazepam), 5-(2-Fluorphenyl)-1-methyl-7-nitro-1*H*-1,4-benzodiazepin-2(3*H*)-on (Flunitrazepam), 7-Chlor-1-(2-diethylaminoethyl)-5-(2-fluorphenyl)-1*H*-1,4-benzodiazepin-2(3*H*)-on (Flurazepam), 7-Chlor-5-phenyl-1-(2,2,2-trifluorethyl)-1*H*-1,4-benzodiazepin-2(3*H*)-on (Halazepam), 10-Brom-11b-(2-fluorphenyl)-2,3,7,11b-tetrahydro[1,3]oxazolo[3,2-d][1,4]benzodiazepin-6(5*H*)-on (Haloxazolam), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 11-Chlor-8,12b-dihydro-2,8-dimethyl-12b-phenyl-4*H*-[1,3]oxazino[3,2-d][1,4]benzodiazepin-4,7(6*H*)-dion (Ketazolam), 1-[4-(3-Hydroxyphenyl)-1-methyl-4-piperidyl]-1-propanon (Ketobemidon), (3*S*,6*S*)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 6-(2-Chlorphenyl)-2-(4-methyl-1-piperazinylmethylen)-8-nitro-2*H-*imidazo[1,2-a][1,4]benzodiazepin-1(4*H*)-on (Loprazolam), 7-Chlor-5-(2-chlorphenyl)-3-hydroxy-1*H*-1,4-benzodiazepin-2(3*H*)-on (Lorazepam), 7-Chlor-5-(2-chlorphenyl)-3-hydroxy-1-methyl-1*H*-1,4-benzodiazepin-2(3*H*)-on (Lormetazepam), 5-(4-Chlorphenyl)-2,5-dihydro-3*H*-imidazo[2,1-*a*]isoindol-5-ol (Mazindol), 7-Chlor-2,3-dihydro-1-methyl-5-phenyl-1*H*-1,4-benzodiazepin (Medazepam), *N*-(3-Chlorpropyl)-α-methylphenethylamin (Mefenorex), Meperidin, 2-Methyl-2-propyltrimethylendicarbamat (Meprobamat), Meptazinol, Metazocin, Methylmorphin, N,α-Dimethylphenethylamin (Metamfetamin), (±)-6-Dimethylamino-4,4-diphenyl-3-heptanon (Methadon), 2-Methyl-3-*o*-tolyl-4(3*H*)-chinazolinon (Methaqualon), Methyl-[2-phenyl-2-(2-piperidyl)acetat] (Methylphenidat), 5-Ethyl-1-methyl-5-phenylbarbitursäure (Methylphenobarbital), 3,3-Diethyl-5-methyl-2,4-piperidindion (Methyprylon), Metopon, 8-Chlor-6-(2-fluorphenyl)-1-methyl-4*H-*imidazo[1,5-*a*][1,4]benzodiazepin (Midazolam), 2-(Benzhydrylsulfinyl)acetamid (Modafinil), 4,5α-Epoxy-17-methyl-7-morphinen-3,6α-diol (Morphin), Myrophin, (±)-*trans*-3-(1,1-Dimethylheptyl)-7,8,10,10α-tetrahydro-1-hydroxy-6,6-dimethyl-6*H-*dibenzo [*b, d*]pyran-9(6α*H*)-on (Nabilon), Nalbuphen, Nalorphin, Narcein, Nicomorphin, 1-Methyl-7-nitro-5-phenyl-1*H*-1,4-benzodiazepin-2(3*H*)-on (Nimetazepam), 7-Nitro-5-phenyl-1*H*-1,4-benzodiazepin-2(3*H*)-on (Nitrazepam), 7-Chlor-5-phenyl-1*H*-1,4-benzodiazepin-2(3*H*)-on (Nordazepam), Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyl-1*H*-1,4-benzodiazepin-2(3*H*)-on (Oxazepam), (*cis-trans*)-10-Chlor-2,3,7,11b-tetrahydro-2-methyl-11b-phenyloxazolo[3,2-*d*][1,4] benzodiazepin-6-(5*H*)-on (Oxazolam), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pernolin), 1,2,3,4,5,6-Hexahydro-6,11-dimethyl-3-(3-methyl-2-butenyl)-2,6-methano-3-benzazocin-8-ol (Pentazocin), 5-Ethyl-5-(1-methylbutyl)-barbitursäure (Pentobarbital), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), 5-Ethyl-5-phenylbarbitursäure (Phenobarbital), α,α-Dimethylphenethylamin (Phentermin), 7-Chlor-5-phenyl-1-(2-propinyl)-1*H*-1,4-benzodiazepin-2(3*H*)-on (Pinazepam), α-(2-Piperidyl)benzhydrylalkohol (Pipradrol), 1'-(3-Cyan-3,3-diphenylpropyl)[1,4'-bipiperidin]-4'-carboxamid (Piritramid), 7-Chlor-1-(cyclopropylmethyl)-5-phenyl-1*H*-1,4-benzodiazepin-2(3*H*)-on (Prazepam), Profadol, Proheptazin, Promedol, Properidin, Propoxyphen, Pseudoephedrin, N-(1-Methyl-2-piperidinoethyl)-N-(2-pyridyl)propionamid, Methyl{3-[4-methoxycarbonyl-4-(*N-*phenylpropanamido)piperidino]propanoat} (Remifentanil), 5-*sec*-Butyl-5-ethylbarbitursäure (Secbutabarbital), 5-Allyl-5-(1-methylbutyl)-barbitursäure (Secobarbital), *N*-{4-Methoxymethyl-1-[2-(2-thienyl)ethyl]-4-piperidyl}propionanilid (Sufentanil), 7-Chlor-2-hydroxy-methyl-5-phenyl-1*H*-1,4-benzodiazepin-2(3*H*)-on (Temazepam), 7-Chlor-5-(1-cyclohexenyl)-1-methyl-1*H*-1,4-benzodiazepin-2(3*H*)-on (Tetrazepam), Ethyl-(2-dimethylamino-1-phenyl-3-cyclohexen-1-carboxylat) (Tilidin (cis und trans)), Tramadol, 8-Chlor-6-(2-chlorphenyl)-1-methyl-4*H*-[1,2,4]triazolo[4,3-a][1,4]benzodiazepin (Triazolam), 5-(1-Methylbutyl)-5-vinylbarbitursäure (Vinylbital), (1 R,2R)-3-(3-Dimethylamino-1-ethyl-2-methyl-propyl)-phenol, (1R, 2R, 4S)-2- [Dimethylamino)methyl-4-(p-fluorbenzyloxy)-1-(m-methoxyphenyl)cyclohexanol, (1R, 2R)-3-(2-Dimethylaminomethyl-cyclohexyl)-phenol, (1S, 2S)-3(3-Dimethylamino-1-ethyl-2-methyl-propyl)-phenol, (2R, 3R)-1-Dimethylamino-3(3-Methoxy-phenyl)-2-methyl-pentan-3-ol, (1RS, 3RS, 6RS)-6-Dimethylaminomethyl-1-(3-methoxy-phenyl)-cyclohexan-1,3-diol, vorzugsweise als Racemat, 3-(2-Dimethylaminomethyl-1-hydroxy-cyclohexyl)-phenyl 2-(4-isobutylphenyl)-propionat, 3-(2-Dimethylaminomethyl-1-hydroxy-cyclohexyl)phenyl 2-(6-methoxy-naphthalen-2-yl)-xpropionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)-phenyl ester, (RR-SS)-2-Hydroxy-4-trifluoromethyl-benzoesäure 3-(2-dimethylaminomethyl-1-hydroxy-cyclohexyl)-phenyl ester, (RR-SS)-4-Chloro-2-hydroxy-benzoesäure 3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäure 3-(2-dimethylaminomethyl-1-hydroxy-cyclohexyl)-phenyl-ester, (RR-SS)-2-Hydroxy-5-nitro-benzoesäure 3-(2-dimethylaminomethyl-1-hydroxy-cyclohexyl)-phenyl ester, (RR-SS)-2',4'-Difluoro-3-hydroxy-biphenyl-4-carbonsäure 3-(2-dimethylaminomethyl-1-hydroxy-cyclohexyl)-phenyl ester sowie entsprechende stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt Hydrochloride.

Die erfindungsgemäße Darreichungsform eignet sich insbesondere zur Erschwerung bzw. Verhinderung des Missbrauchs eines opioiden Wirkstoffes ausgewählt aus der Gruppe umfassend Oxycodon, Diamorphin, Ethylmorphin, Hydrocodon, Oxymorphon, Hydromorphon, Morphin, Tramadol und deren physiologisch verträgliche Derivate oder Verbindungen, vorzugsweise deren Salze und Solvate, vorzugsweise deren Hydrochloride, physiologisch verträgliche Enantiomere, Stereoisomere, Diastereomere und Racemate und deren physiologisch verträglichen Derivate, vorzugsweise Ether, Ester oder Amide.

Weiterhin eignet sich die erfindungsgemäße Darreichungsform insbesondere zur Erschwerung bzw. Verhinderung des Missbrauchs eines opioiden Wirkstoffes ausgewählt aus der Gruppe umfassend (1 R, 2R)-3-(3-Dimethylamino-1-ethyl-2-methyl-propyl)-phenol, (2R, 3R)-1-Dimethylamino-3-(3-methoxy-phenyl)-2-methyl-pentan-3-ol, (1RS, 3RS, 6RS)-6-Dimethylaminomethyl-1-(3-methoxy-phenyl)-cyclohexane-1,3-diol, (1R, 2R)-3-(2-Dimethylaminonethyl-cyclohexyl)-phenol, deren physiologisch verträglichen Salze, vorzugsweise Hydrochloride, Phosphate, Maleate, physiologisch verträgliche Enantiomere, Stereoisomere, Diastereomere und Racemate und deren physiologisch verträglichen Derivate, vorzugsweise Ether, Ester oder Amide.

Diese Verbindungen bzw. deren Herstellungsverfahren sind in der EP-A-693475 bzw. EP-A-780369 beschrieben.

Zur Erzielung der notwendigen Bruchfestigkeit der Partikel der erfindungsgemäßen Darreichungsform werden mindestens ein synthetisches oder natürliches Polymer (C) mit einer Bruchfestigkeit, gemessen nach der in der vorliegenden Anmeldung offenbarten Methode, von mindestens 500 N eingesetzt. Bevorzugt wird hierfür mindestens ein Polymeres ausgewählt aus der Gruppe umfassend Polyalkylenoxide, vorzugsweise Polymethylenoxide, Polyethylenoxide, Polypropylenoxide; Polyethylene, Polypropylene, Polyvinylchloride, Polycarbonate, Polystyrole, Polyacrylate, deren Copolymerisate und Mischungen aus mindestens zwei der genannten Polymeren eingesetzt. Bevorzugt sind hochmolekulare, thermoplastische Polyalkylenoxide. Besonders bevorzugt sind hochmolekulare Polyethylenoxide mit einem Molekulargewicht von mindestens 0,5 Mio., vorzugsweise von mindestens 1 Mio., besonders bevorzugt 1 Mio. bis 15 Mio., ganz besonders bevorzugt von 1 bis 10 Mio., bestimmt durch rheologische Messungen. Diese Polymeren weisen eine Viskosität bei 25 °C von 4500 bis 17600 cP, gemessen an einer 5 Gew.% wässrigen Lösung mit Hilfe eines Brookfield Viskosimeter, Model RVF (Spindel Nr. 2 / Rotationsgeschwindigkeit 2 rpm), von 400 bis 4000 cP, gemessen an einer 2 Gew.% wässrigen Lösung mit Hilfe des genannten Viskosimeters (Spindel Nr. 1 bzw. 3 / Rotationsgeschwindigkeit 10 rpm) bzw. von 1650 bis 10000 cP, gemessen an einer 1 Gew.% wässrigen Lösung mit Hilfe des genannten Viskosimeters (Spindel Nr. 2 /Rotationsgeschwindigkeit 2 rpm) auf.

Die Polymeren werden bevorzugt als Pulver eingesetzt. Sie können in Wasser löslich sein.

Des weiteren können zusätzlich zur Erzielung der notwendigen Bruchfestigkeit der Partikel der erfindungsgemäßen Darreichungsform mindestens ein natürliches halbsynthetisches oder synthetisches Wachs (D) mit einer Bruchfestigkeit, gemessen nach der in der vorliegenden Anmeldung offenbarten Methode, von mindestens 500 N eingesetzt werden. Bevorzugt sind die Wachse mit einem Erweichungspunkt von mindestens 50°C, besonders bevorzugt von 60°C. Besonders bevorzugt sind Carnaubawachs und Bienenwachs, insbesondere Carnaubawachs. Carnaubawachs ist ein natürliches Wachs, das aus den Blättern der Carnaubapalme gewonnen wird und einen Erweichungspunkt von wenigstens 80°C aufweist. Beim zusätzlichen Einsatz der Wachskomponente wird diese zusammen mit wenigstens einem Polymeren (C) in solchen Mengen eingesetzt, dass die Partikel der Darreichungsform eine Bruchfestigkeit von mindestens 500 N aufweisen.

Vorzugsweise wird die Komponente (C) in einer Menge von 35 bis 99,9 Gew.%, besonders bevorzugt von wenigstens 40 Gew.%, ganz besonders bevorzugt von 40 bis 70 Gew.%, bezogen auf das Gesamtgewicht der Darreichungsform, eingesetzt.

Als Sprengmittel (E) können physiologisch verträgliche Sprengmittel verwendet werden, wie sie zur Herstellung von pharmazeutischen Darreichungsformen zur Anwendung kommen. Vorzugsweise wird als Sprengmittel (E) wenigstens ein Sprengmittel ausgewählt aus der Gruppe umfassend vernetzte Natriumcarboxymethylcellulose (Crosscamellose), modifizierte Maisstärke, Natriumcarboxymethylstärke, vernetztes Polyvinylpyrrolidon (Crosspovidon) eingesetzt. Die erfindungsgemässen Darreichungsformen enthalten vorzugsweise 0,5 bis 25 Gew.%, besonders bevorzugt 1 bis 10 Gew.%, bezogen auf das Gesamtgewicht der Darreichungsform, wenigstens eines Sprengmittels (E).

Vorzugsweise wird das Sprengmittel pulverförmig eingesetzt und liegt in der erfindungsgemässen Darreichungsform in den Partikeln und/oder auf den Partikeln und/oder lose verteilt neben den Partikeln vor. Vorzugsweise liegt das Sprengmittel (E) zumindest teilweise als Formulierungskomponente in den Partikeln der erfindungsgemässen Darreichungsform und/oder zumindest teilweise als Umhüllung der Partikel, vorzugsweise in einem Überzug der Partikel. Das Sprengmittel (E) ist zumindest teilweise mit den Partikeln der Darreichungsform vermischt. Ganz besonders bevorzugt ist das Sprengmittel sowohl als Formulierungskomponente in den Partikeln als auch als Umhüllungskomponente um die Partikel vorhanden.

Als weitere Hilfsstoffe (B) können die üblichen für die Formulierung von festen Darreichungsformen bekannten, vorzugsweise temperaturbeständige Hilfsstoffe (B1) verwendet werden. Vorzugsweise sind dies Weichmacher, Füllstoffe, Antioxidantien und/oder Redoxstabilisatoren.

Als Antioxidantien eignen sich vorzugsweise Ascorbinsäure, [alpha]-Tocopherol, Butylhydroxyanisol, Butylhydroxytoluol, Salze der Ascorbinsäure, Ascorbylpalmitat, Monothioglyzerin, phosphorige Säure, Vitamin C, Vitamin E und dessen Derivate wie Vitamin E-succinat oder Vitamin E-palmitat und/oder Natriumbisulfit, besonders bevorzugt Butylhydroxytoluol (BHT) oder Butylhydroxyanisol (BHA) und/oder α-Tocopherol.

Das Antioxidanz wird vorzugsweise in Mengen von 0,01 bis 10 Gew.%, vorzugsweise 0,03 bis 5 Gew.%, bezogen auf das Gesamtgewicht der Darreichungsform, eingesetzt.

Als Redoxstabilisatoren, wie beispielsweise Chelatbildner, eignen sich vorzugsweise Zitronensäure, EDTA (Ethylendiamintetraessigsäure), Maleinsäure und Fumarsäure.

Als Weichmacher vorzugsweise in einer Menge von 5 - 20 Gew.%, werden vorzugsweise Polyalkylenglykole, vorzugsweise Polyethylenglykole, Fettsäuren, Fettsäureester, Wachse und/oder mikrokristalline Wachse eingesetzt.

Als Füllstoffe in den Partikeln der erfindungsgemässen Darreichungsform können vorzugsweise Methylcellulose, Hydroxypropylcellulose, Hydroxypropylmethylcellulose, Calciumdihydrogenphosphat und/oder Tricalciumphosphat mitverwendet werden.

Als Zusatzstoffe bzw. Hilfsstoffe (B2), die nicht Bestandteile der Partikel sind, können Füllstoffe eingesetzt werden. Vorzugsweise sind die Zusatzstoffe Bestandteile der Partikel und/oder sind bevorzugt Geschmacksverbesserer oder Gleitmittel.

Als Gleitmittel können Siliciumdioxid, Stearinsäure, Talkum, Fettsäureester, Zuckerester und/oder Magnesiumstearat verwendet werden.

Als Geschmacksverbesserer können Aromazusätze, Brausezusätze, Zucker, Zuckeralkohole oder Zuckeraustauschstoffe mitverwendet werden

Als Füllstoffe können mikrokristalline Cellulose, Calciumdihydrogenphosphat, Zuckeralkohole wie Mannitol, Zucker wie Lactose, Pulvercellulose, Collidon und/oder Polyvinylpyrrolidon eingesetzt werden.

Die erfindungsgemässen, multipartikulären Darreichungsformen zeichnen sich dadurch aus, dass sie aufgrund ihrer Härte mit Hilfe von üblichen, einem Missbraucher zur Verfügung stehenden Zerkleinerungsmitteln, wie Mörser und Pistill, nicht zu pulverisieren sind. Ein parenteraler, insbesondere intravenöser oder nasaler Missbrauch ist dadurch erschwert bzw. verhindert. Um jedoch jeden möglichen Missbrauch der erfindungsgemässen Darreichungsformen vorzubeugen, können die erfindungsgemässen Darreichungsformen in einer bevorzugten Ausführungsform als Hilfsstoffe (B3) weitere Missbrauchs-erschwerende bzw. -verhindernde Mittel enthalten.

So kann die erfindungsgemäße, multipartikuläre Darreichungsform, die neben einem oder mehreren Wirkstoffen mit Missbrauchspotential, mindestens einem härtebildenden Polymer (C) wenigstens ein Sprengmittel (E), ggf. mindestens ein Wachs (D), ggf. weitere Hilfsstoffe (B1, B2) noch wenigstens eine der nachfolgenden Komponenten (a)-(e) als Hilfsstoffe (B3) aufweisen:
(a) wenigstens einen zumindest den Nasenraum reizenden Stoff,
(b) wenigstens einen Antagonisten für jeden der in der Darreichungsform vorhandenen Wirkstoffe mit Missbrauchspotential,
(c) wenigstens ein Emetikum,
(d) wenigstens einen Farbstoff als aversives Mittel,
(e) wenigstens einen Bitterstoff.

Die Komponenten (a) bis (e) sind jeweils für sich allein zusätzlich zur Sicherung der erfindungsgemäßen Darreichungsform gegen Missbrauch geeignet. So eignet sich die Komponente (a) bevorzugt zur Sicherung gegen nasalen und/oder parenteralen, vorzugsweise intravenösen, Missbrauch, die Komponente (b) bevorzugt gegen nasalen und/oder parenteralen, besonders bevorzugt intravenösen, Missbrauch, die Komponente (c) vorzugsweise gegen parenteralen, besonders bevorzugt intravenösen, und/oder oralen und/oder nasalen Missbrauch, die Komponente (d) als visuelles Abschreckungsmittel gegen oralen oder parenteralen Missbrauch und die Komponente (e) gegen oralen oder nasalen Missbrauch. Durch die erfindungsgemäße Mitverwendung von wenigstens einer der vorstehend genannten Komponenten, gelingt es, bei erfindungsgemäßen Darreichungsformen noch effektiver den Missbrauch zu erschweren.

In einer Ausführungsform kann die erfindungsgemäße Darreichungsform auch zwei oder mehrere der Komponenten (a)-(e) in einer Kombination aufweisen, vorzugsweise (a) und ggf. (c) und/oder (e) und/oder (d) bzw. (a) und ggf. (c) und/oder (d) und/oder (e).

In einer weiteren Ausführungsform kann die erfindungsgemäße Darreichungsform sämtliche Komponenten (a)-(e) aufweisen.

Sofern die erfindungsgemäße Darreichungsform gegen Missbrauch die Komponente (a) umfasst, kommen als den Nasen- und/oder Rachenraum reizende Stoffe erfindungsgemäß sämtliche Stoffe in Betracht, die bei entsprechender Applikation über den Nasen- und/oder Rachenraum eine Reaktion des Körpers hervorrufen, die entweder für den Missbraucher so unangenehm ist, dass er die Applikation nicht weiter fortsetzen will oder kann, z.B. ein Brennen, oder die auf physiologische Art und Weise einer Aufnahme des entsprechenden Wirkstoffes entgegenwirken, z.B. über eine vermehrte nasale Sekretbildung oder Niesen. Diese üblicherweise den Nasen- und/oder Rachenraum reizenden Stoffe können auch bei parenteraler, insbesondere intravenöser, Applikation ein sehr unangenehmes Gefühl bis hin zu unerträglichen Schmerzen verursachen, so dass der Missbraucher die Einnahme nicht länger fortsetzen will oder kann.

Besonders geeignete, den Nasen- und/oder Rachenraum reizende Stoffe sind solche Stoffe, die ein Brennen, einen Juckreiz, einen Niesreiz, eine vermehrte Sekretbildung oder eine Kombination mindestens zweier dieser Reize verursachen. Entsprechende Stoffe und deren üblicherweise einzusetzenden Mengen sind dem Fachmann an sich bekannt oder können durch einfache Vorversuche ermittelt werden.

Der den Nasen- und/oder Rachenraum reizende Stoff der Komponente (a) basiert vorzugsweise auf einem oder mehreren Inhaltsstoffen oder einem oder mehreren Pflanzenteilen wenigstens einer Scharfstoffdroge.

Entsprechende Scharfstoffdrogen sind dem Fachmann an sich bekannt und werden beispielsweise in "Pharmazeutische Biologie - Drogen und ihre Inhaltsstoffe" von Prof. Dr. Hildebert Wagner, 2., bearbeitete Auflage, Gustav Fischer Verlag, Stuttgart-New York, 1982, Seiten 82 ff., beschrieben.

Unter einer Darreichungseinheit der erfindungsgemäßen, multipartikulären Darreichungsform wird eine separate bzw. separierbare Dosis, wie z. B. eine Kapselfüllung der erfindungsgemäßen Darreichungsform, verstanden.

Vorzugsweise kann der erfindungsgemäßen Darreichungsform als Komponente (a) einer oder mehrere Inhaltsstoffe wenigstens einer Scharfstoffdroge, ausgewählt aus der Gruppe bestehend aus Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhizoma, besonders bevorzugt aus der Gruppe bestehend aus Capsici Fructus (Paprika), Capsici Fructus acer (Cayennepfeffer) und Piperis nigri Fructus (Pfeffer), hinzugefügt werden.

Bei den Inhaltsstoffen der Scharfstoffdrogen handelt es sich bevorzugt um o-Methoxy(Methyl)-phenol-Verbindungen, Säureamid-Verbindungen, Senföle oder Sulfidverbindungen oder um davon abgeleiteten Verbindungen.

Besonders bevorzugt ist wenigstens ein Inhaltsstoff der Scharfstoffdrogen ausgewählt aus der Gruppe bestehend aus Myristicin, Elemicin, Isoeugenol, α-Asaron, Safrol, Gingerolen, Xanthorrhizol, Capsaicinoiden, vorzugsweise Capsaicin, Capsaicin- Derivate, wie N-vanillyl -9E-octadecenamid, Dihydrocapsaicin, Nordihydrocapsaicin, Homocapsaicin, Norcapsaicin, und Nomorcapsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmercaptosenföl oder Methylsulfonylsenföl, und von diesen Inhaltsstoffen abgeleiteten Verbindungen.

Vorzugsweise kann die erfindungsgemäße Darreichungsform die Pflanzenteile der entsprechenden Scharfstoffdrogen in einer Menge von 0,01 bis 30 Gew.-%, besonders bevorzugt 0,1 bis 0,5 Gew.-%, jeweils bezogen auf das Gesamtgewicht einer Darreichungseinheit bzw. Dosiseinheit, enthalten.
Kommen ein oder mehrere Inhaltsstoffe entsprechender Scharfstoffdrogen zum Einsatz, beträgt deren Menge bevorzugt 0,001 bis 0,005 Gew.-%, bezogen auf das Gesamtgewicht der Darreichungseinheit bzw. Dosiseinheit.

Des weiteren kann die erfindungsgemäße Darreichungsform zur Vorbeugung und Sicherung gegen Missbrauch die Komponente (b) aufweisen, nämlich einen oder mehrere Antagonisten für den Wirkstoff bzw. die Wirkstoffe mit Missbrauchspotential, wobei die Antagonistenmenge vorzugsweise räumlich getrennt von den übrigen Bestandteilen der erfindungsgemäßen Darreichungsform vorliegen und keine Wirkung bei bestimmungsgemäßer Verwendung entfalten.

Geeignete Antagonisten zur Verhinderung des Mißbrauchs der Wirkstoffe sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Sofern der in der Darreichungsform vorliegende Wirkstoff ein Opioid ist, kommt als Antagonist bevorzugt ein Antagonist ausgewählt aus der Gruppe umfassend Naloxon, Naltrexon, Nalmefen, Nalid, Nalmexon, Nalorphin oder Naluphin, jeweils ggf. in Form einer entsprechenden physiologisch verträglichen Verbindung, insbesondere in Form einer Base, eines Salzes oder Solvates, zum Einsatz. Vorzugsweise werden die entsprechenden Antagonisten, sofern eine Ausrüstung mit der Komponente (b) vorgesehen ist, in einer Menge von ≥ 1 mg, besonders bevorzugt in einer Menge von 3 bis 100 mg, ganz besonders bevorzugt in einer Menge von 5 bis 50 mg auf pro Darreichungsform, d.h. pro Dosiseinheit eingesetzt.

Weist die erfindungsgemäße Darreichungsform als Wirkstoff ein Stimulanz auf, ist der Antagonist bevorzugt ein Neuroleptikum, vorzugsweise wenigstens eine Verbindung ausgewählt aus der Gruppe bestehend aus Haloperidol, Promethacin, Fluphenazin, Perphenazin, Levomepromazin, Thioridazin, Perazin, Chlorpromazin, Chlorprothixin, Zuclopentixol, Flupentexol, Prothipendyl, Zotepin, Benperidol, Pipamperon, Melperon und Bromperidol.

Vorzugsweise weist die erfindungsgemäße Darreichungsform diese Antagonisten in einer üblichen, dem Fachmann bekannten therapeutischen Dosierung, besonders bevorzugt in einer gegenüber der üblichen Dosierung verdoppelten bis verdreifachten Menge pro Dosiseinheit auf.

Sofern die Kombination zur Vorbeugung und Sicherung der erfindungsgemäßen Darreichungsform gegen Missbrauch die Komponente (c) umfasst, kann sie wenigstens ein Emetikum aufweisen, das vorzugsweise in einer räumlich getrennten Anordnung von den übrigen Komponenten der erfindungsgemäßen Darreichungsform vorliegen und bei bestimmungsgemäßer Anwendung keine Wirkung im Körper entfalten sollte.

Geeignete Emetika zur Verhinderung des Missbrauchs eines Wirkstoffs sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

In der erfindungsgemäßen Darreichungsform kann bevorzugt ein Emetikum auf Basis eines oder mehrerer Inhaltsstoffe von Radix Ipecacuanhae (Brechwurzel), vorzugsweise auf Basis des Inhaltsstoffes Emetin, in Betracht, wie sie z.B. in "Pharmazeutische Biologie - Drogen und ihre Inhaltsstoffe" von Prof. Dr. Hildebert Wagner, 2., bearbeitete Auflage, Gustav Fischer Verlag, Stuttgart, New York 1982 beschrieben werden.

Vorzugsweise kann die erfindungsgemäße Darreichungsform als Komponente (c) das Emetikum Emetin aufweisen, bevorzugt in einer Menge von ≥ 3 mg, besonders bevorzugt ≥ 10 mg und ganz besonders bevorzugt in einer Menge von ≥ 20 mg pro Darreichungsform, d.h. Dosiseinheit.

Ebenfalls bevorzugt kann als Emetikum Apomorphin in der erfindungsgemäßen Missbrauchssicherung zum Einsatz kommen, vorzugsweise in einer Menge von vorzugsweise ≥ 3 mg, besonders bevorzugt ≥ 5 mg und ganz besonders bevorzugt ≥ 7 mg pro Dosiseinheit.

Sofern die erfindungsgemäße Darreichungsform die Komponente (d) als weiteren missbrauchsverhindemden Hilfsstoff enthält, so wird durch den Einsatz eines solchen Farbstoffes, insbesondere bei dem Versuch, den Wirkstoff für eine parenterale, vorzugsweise intravenöse Applikation, zu extrahieren, eine intensive Farbgebung einer entsprechenden wässrigen Lösung hervorgerufen, die zur Abschreckung beim potentiellen Missbraucher führen kann. Auch ein oraler Missbrauch, der üblicherweise über eine wässrige Extraktion des Wirkstoffes eingeleitet wird, kann durch diese Farbgebung verhindert werden. Geeignete Farbstoffe sowie die für die notwendige Abschreckungswirkung erforderlichen Mengen sind der WO 03/015531 zu entnehmen.

Sofern die erfindungsgemäße Darreichungsform als zusätzlichen Missbrauchsverhindernden Hilfsstoff die Komponente (e) enthält, so wird durch diesen Zusatz von wenigstens einem Bitterstoff durch die damit eintretende Geschmacksverschlechterung der Darreichungsform der orale und/oder nasale Missbrauch zusätzlich verhindert.

Geeignete Bitterstoffe sowie die für den Einsatz wirksamen Mengen sind der US-2003/0064099 A1 zu entnehmen. Vorzugsweise eignen sich als Bitterstoffe Aromaöle, vorzugsweise Pfefferminzöl, Eukalyptusöl, Bittermandelöl, Menthol, Fruchtaromastoffe, vorzugsweise Aromastoffe von Zitronen, Orangen, Limonen, Grapefruit oder Mischungen davon, und/oder Denatonium-Benzoat (Bitrex®). Besonders bevorzugt ist Denatonium-Benzoat.

Die erfindungsgemäße feste Darreichungsform eignet sich zur oralen Einnahme. Die erfindungsgemäße, multipartikuläre Darreichungsform kann durch unterschiedliche Verfahren hergestellt werden, die nachfolgend näher erläutert werden; die Erfindung betrifft auch Darreichungsformen, welche nach einem dieser Verfahren erhältlich sind:

Das Verfahren zur Herstellung der erfindungsgemäßen Darreichungsform umfasst bevorzugt folgende Schritte:
(a) Mischen der Komponente (A), ggf. (B), (C), ggf. (D) und ggf. zumindest teilweise (E);
(b) ggf. Vorformen der aus Schritt (a) erhaltenen Mischung, vorzugsweise unter Wärme- und/oder Krafteinwirkung auf die aus (a) erhaltene Mischung, wobei die zugeführte Wärmemenge bevorzugt nicht dazu ausreicht, die Komponente (C) bis zu ihrem Erweichungspunkt zu erwärmen;
(c) Härten der Mischung unter Wärme- und Krafteinwirkung, wobei die Wärmezufuhr während und/oder vor der Krafteinwirkung erfolgen kann und die zugeführte Wärmemenge dazu ausreicht, die Komponente (C) wenigstens bis zu ihrem Erweichungspunkt zu erwärmen;
(d) Vereinzeln der gehärteten Mischung;
(e) ggf. Formen zu der Darreichungsform; und
(f) ggf. Beschichten mit einem Überzug enthaltend die Komponente (E) und/oder Mischen mit der Komponente (E) und ggf. Zusatzstoffen (B2).

Die Wärme kann direkt oder mit Hilfe von Ultraschall zugeführt werden. Die Krafteinwirkung und/oder das Formen der Darreichungsform kann beispielsweise mit Hilfe von geeigneten Extrudern erfolgen, insbesondere mit Doppelschneckenextrudern (Doppelwalzenextrudern) oder Planetwalzenextrudern.

Die folgenden Verfahrensvarianten sind besonders bevorzugt:

### Verfahrensvariante 1:

In dieser Ausführungsform wird die erfindungsgemäße Darreichungsform vorzugsweise mit Extruder-Einsatz hergestellt, indem vorzugsweise die Komponenten (A), ggf. (B), (C), die ggf. vorhandene Komponente (D) und ggf. die zumindest teilweise mitverwendete Komponente (E) gemischt werden und die resultierende Mischung ggf. nach einer Granulierung zu der Darreichungsform unter vorangehender oder gleichzeitiger Wärmeeinwirkung durch Krafteinwirkung geformt wird.

Diese Erwärmung und Krafteinwirkung zur Herstellung der Darreichungsform erfolgt mit Hilfe eines Extruders.

Die Mischung der Komponenten (A), ggf. (B), (C) und ggf. (D) erfolgt in einem dem Fachmann bekannten Mischgerät. Das Mischgerät kann beispielsweise ein Wälzmischer, Schüttelmischer, Schermischer oder Zwangsmischer sein.

Die Krafteinwirkung erfolgt solange, bis die Darreichungsform eine Bruchhärte von mindestens 500 N, erreicht hat.

Die Granulierung kann durch Feuchtgranulation oder Schmelzgranulation in bekannten Granulatoren durchgeführt werden.

Besonders bevorzugt wird diese erfindungsgemäße Variante zur Herstellung der erfindungsgemäßen Darreichungsformen so durchgeführt, dass man
a) die Komponenten (A), ggf. (B), (C), die gegebenenfalls vorhandene Komponente (D) und ggf. zumindest einen Teil der Komponente (E) mischt,
b) die resultierende Mischung im Extruder mindestens bis zum Erweichungspunkt der Komponente (C) erwärmt und unter Krafteinwirkung durch die Austrittsöffnung des Extruders extrudiert,
c) das noch plastische Extrudat vereinzelt und ggf. zur multipartikulären Darreichungsform formt und ggf. mit der Komponente (E) mischt und/oder umhüllt.

Vorzugsweise kann das Mischen der Komponenten gemäß dem Verfahrensschritt a) ebenfalls bereits in dem Extruder erfolgen.

Vorzugsweise wird vor dem Abmischen mit den weiteren Komponenten die Komponente (C) und die gegebenenfalls vorhandene Komponente (D) erfindungsgemäß mit einem Antioxidanz versehen. Dies kann durch Vermischen der beiden Komponenten (C) und dem Antioxidanz erfolgen, vorzugsweise indem das Antioxidanz in einem leicht flüchtigen Lösungsmittel aufgelöst oder suspendiert und diese Lösung oder Suspension mit der Komponente (C) und der gegebenenfalls vorhandenen Komponente (D) homogen vermischt und das Lösungsmittel durch Trocknung, vorzugsweise unter Inertgasatmosphäre, entfernt wird.

Die im Extruder bis mindestens zum Erweichungspunkt der Komponente (C) erwärmte, vorzugsweise schmelzflüssige Mischung aus dem Extruder wird durch eine Düse mit mindestens einer Bohrung extrudiert.

Zur Durchführung des erfindungsgemäßen Verfahrens ist der Einsatz geeigneter Extruder erforderlich, bevorzugt Schneckenextruder (Walzenextruder), wobei Extruder, die mit zwei Schnecken (Walzen) ausgerüstet sind, besonders bevorzugt sind. Bevorzugt weisen die Schnecken exzentrische Spritzen auf und der Extruder ist vorzugsweise mit einem Verdrängerkegel ausgerüstet.

Die Extrusion wird vorzugsweise so durchgeführt, dass die Aufweitung der Extrusionsstränge infolge der Extrusion vorzugsweise höchstens 50% beträgt, d.h. dass beispielsweise bei Verwendung einer Extrusionsdüse mit 1 mm Bohrungen die extrudierten Stränge einen Durchmesser von höchstens 1,5 mm aufweisen. Bevorzugter beträgt die Strangaufweitung höchstens 40%, noch bevorzugter höchstens 35%, am bevorzugtesten höchstens 30% und insbesondere höchstens 25%. Es wurde überraschend festgestellt, dass bei zu starker mechanischer Beanspruchung des extrudierten Materials im Extruder eine erhebliche Strangaufweitung erfolgt, welche in unerwünschten Unregelmäßigkeiten der Eigenschaften, insbesondere der mechanischen Eigenschaften des extrudierten Strangs resultiert.

Vorzugsweise weist der Extruder mindestens zwei Temperaturzonen auf, wobei in der ersten Zone, die sich an eine Einzugs- und ggf. Mischzone anschließt, das Aufheizen der Mischung bis mindestens zum Erweichungspunkt der Komponente (C) stattfindet.

Nach Erwärmung bis mindestens zum Erweichungspunkt der Komponente (C) wird die aufgeschmolzene Mischung mit Hilfe der Schnecken gefördert, weiter homogenisiert, komprimiert bzw. kompaktiert, so dass sie unmittelbar vor dem Austritt aus der Extruderdüse einen Mindestdruck von 5 bar, vorzugsweise mindestens 10 bar aufweist, und durch die Düse als Extrusionsstrang oder Extrusionsstränge, je nachdem wie viele Bohrungen die Düse aufweist, extrudiert. Vorzugsweise weist die Düse mehrere Bohrungen auf. Die Düsengeometrie bzw. die Geometrie der Bohrungen ist frei wählbar. So kann die Düse bzw. die Bohrungen einen runden, oblongen oder ovalen Querschnitt aufweisen, wobei der runde Querschnitt vorzugsweise einen Durchmesser von 0,1 mm bis 5 mm aufweist. Vorzugsweise hat die Düse bzw. die Bohrungen einen runden Querschnitt. Der Mantel des erfindungsgemäß zum Einsatz kommenden Extruders kann beheizt oder gekühlt werden. Die entsprechende Temperierung, d.h. Beheizung oder Kühlung, richtet sich danach, dass die zu extrudierende Mischung mindestens eine Durchschnittstemperatur (Produkttemperatur) entsprechend der Erweichungstemperatur der Komponente (C) aufweist und nicht über eine Temperatur steigt, bei welcher die zur verarbeitende physiologisch wirksame Substanz (A) Schaden nehmen kann. Vorzugsweise wird die Temperatur der zu extrudierenden Mischung unter 180°C, vorzugsweise unter 150°C, aber mindestens auf die Erweichungstemperatur der Komponente (C), eingestellt.

Nach der Extrusion der geschmolzenen Mischung und gegebenenfalls Kühlung der extrudierten Stränge erfolgt vorzugsweise eine Vereinzelung der Extrudate. Diese Vereinzelung kann vorzugsweise durch Zerschneiden der Extrudate mittels mitlaufender oder rotierender Messer, Wasserstrahlschneider, Drähten, Klingen oder mit Hilfe von Laserschneidern durchgeführt werden. An die Vereinzelungen schließt sich vorzugsweise eine Pelletisierung an.

Die Krafteinwirkung im Extruder auf die zumindest plastifizierte Mischung wird durch Steuerung der Umdrehungsgeschwindigkeit der Fördereinrichtung im Extruder und deren Geometrie und durch die Dimensionierung der Austrittsöffnung so eingestellt, dass sich im Extruder vorzugsweise vor der unmittelbaren Extrudierung der plastifizierten Mischung der dafür notwendige Druck aufbaut. Durch einfache Vorversuche können für die jeweilige Zusammensetzung die notwendigen Extrusionsparameter festgestellt werden, die zu einer Darreichungsform mit einer Bruchfestigkeit von mindestens 500 N führen.

Zur Extrusion eignet sich beispielsweise ein Doppelschneckenextruder der Fa. Leistritz (Nürnberg) vom Typ ZSE 18 HP 40D, vorzugsweise mit Schnecken, die mit exzentrischen Schneckenenden ausgerüstet sind. Als Düse kann eine beheizbare Düsenplatte mit 8 Bohrungen à 1,0 mm Durchmesser dienen. Die Extrusionparameter können beispielsweise auf folgende Werte eingestellt werden: Schneckendrehzahl: 150 Upm; Durchsatz: 2 kg/h; Produkttemperatur: 50 bis 140!C, vorzugsweise 80 bis 140°C, besonders bevorzugt 100 bis 140°C, ganz besonders bevorzugt 110 bis 140!C und entsprechende Manteltemperatur.

### Verfahrensvariante 2:

Bei dieser Verfahrensvariante zur Herstellung der erfindungsgemäßen Darreichungsform erfolgt die Energiezufuhr mit Hilfe von Ultraschall.

Dazu wird zunächst eine homogene Mischung aus wenigstens der Komponente (A), der Komponente (C), ggf. der Komponente (D) und ggf. eines Teils der Komponente (E) hergestellt. Dieser Mischung können noch weitere Hilfsstoffe (B1), wie zum Beispiel Füllstoffe, Weichmacher, Gleitmittel oder Farbstoffe, zugemischt werden. Vorzugsweise wird als Weichmacher ein niedermolekulares Polyethylenglykol verwendet.

Das Mischen kann mit Hilfe von üblichen Mischern durchgeführt werden. Beispielsweise sind als Mischer Wälzmischer, die auch als Fall-, Trommel- oder Rotationsmischer bekannt sind, Containermischer, Fassmischer (Rhönradmischer oder Taumelmischer) oder Schüttelmischer, Schermischer, Zwangsmischer, Pflugscharmischer, Planeten-Mischkneter, Z-Kneter, Sigma-Kneter, Fluidmischer oder Intensivmischer geeignet.

Die Auswahl des geeigneten Mischers hängt unter anderem von der Rieselfähigkeit und Kohäsionskräfte des Mischgutes ab.

Die Mischung wird anschließend einer Formgebung unterworfen. Vorzugsweise während oder nach der Ultraschalleinwirkung erfolgt die Formgebung der Mischung.

Bei der Ultraschalleinwirkung ist es besonders bevorzugt, dass ein direkter Kontakt zwischen der Mischung und der Sonotrode des Ultraschallgerätes vorhanden ist.

Bei der Ultraschalleinwirkung sollte eine Frequenz von 1 kHz bis 2 MHz, vorzugsweise 15 bis 40 kHz, eingehalten werden. Die Dauer der Ultraschalleinwirkung sollte solange erfolgen, bis eine Erweichung des Polymers (C) erreicht wird. Vorzugsweise wird dies innerhalb von wenigen Sekunden, besonders bevorzugt innerhalb von 0,1 bis 5 Sekunden, vorzugsweise 0,5 bis 3 Sekunden, erreicht.

Bevor die Formgebung durchgeführt wird, kann nach dem Mischvorgang eine Granulierung der Mischung erfolgen, wonach die daraus resultierenden Granulate unter Ultraschalleinwirkung und Krafteinwirkung zu der Darreichungsform, wie Tabletten, geformt werden.

Die Granulierung kann in den dem Fachmann bekannten Maschinen und Apparaturen durchgeführt werden.

Sofern die Granulierung als Feuchtgranulierung durchgeführt wird, können als Granulierflüssigkeit Wasser oder wässrige Lösungen, wie z.B. Ethanol/Wasser oder Isopropanol/Wasser, eingesetzt werden.

Die Mischung oder die daraus hergestellten Granulate können vorzugsweise auch zur weiteren Formgebung einer Schmelzextrusion unterworfen werden, wobei die Mischung unter Ultraschalleinwirkung und Krafteinwirkung zum Schmelzen gebracht wird und durch Düsen anschließend extrudiert wird. Die so gewonnenen Stränge werden mit Hilfe von bekannten Vorrichtungen auf die gewünschte Länge vereinzelt. Die so vereinzelten Formlinge können noch gegebenenfalls pelletisiert werden, um die erfindungsgemäße, multipartikuläre Darreichungsform mit einer Mindestbruchfestigkeit von 500 N zu erhalten. Die Partikel werden vorzugsweise noch mit der ggf. restlichen Menge an Sprengmittel und ggf. Zusatzstoffen (B2) versehen, bevor sie zu einer Dosiseinheit, z. B. in Kapseln, abgefüllt oder zu einer Tablette verpresst werden.

Bei der Ultraschallanwendung sind geeignete Parameter zur Plastifizierung eine Frequenz: 20 kHz; Amplitude: 50%. Weiterhin sollte eine Kraft: 250 N aufgebracht werden. Die Ultraschall- und Krafteinwirkung mit Hilfe der Sonotrode kann beispielsweise 0,5 Sekunden dauern, wobei Ultraschall- und Krafteinwirkung vorzugsweise gleichzeitig erfolgen.

### Verfahrensvariante 3:

Bei dieser Verfahrensvariante zur Herstellung der erfindungsgemäßen, multipartikulären Darreichungsform werden die Komponenten (A), (C), ggf. (D), ggf. zumindest ein Teil des Sprengmittels (E) und ggf. vorhandene Hilfsstoffe (B1), wie Antioxidantien und Weichmacher mit Hilfe eines Planetwalzen-Extruders zu der erfindungsgemäßen Darreichungsform verarbeitet.

Planetwalzen-Extruder sind bekannt und u.a. ausführlich im Handbuch der Kunststoff-Extrusionstechnik I (1989) "Grundlagen" im Kapitel 1.2 "Klassifizierung von Extrudern" Seiten 4 bis 6 beschrieben. Die entsprechende Beschreibung wird hiermit als Referenz eingeführt und gilt als Teil der Offenbarung.

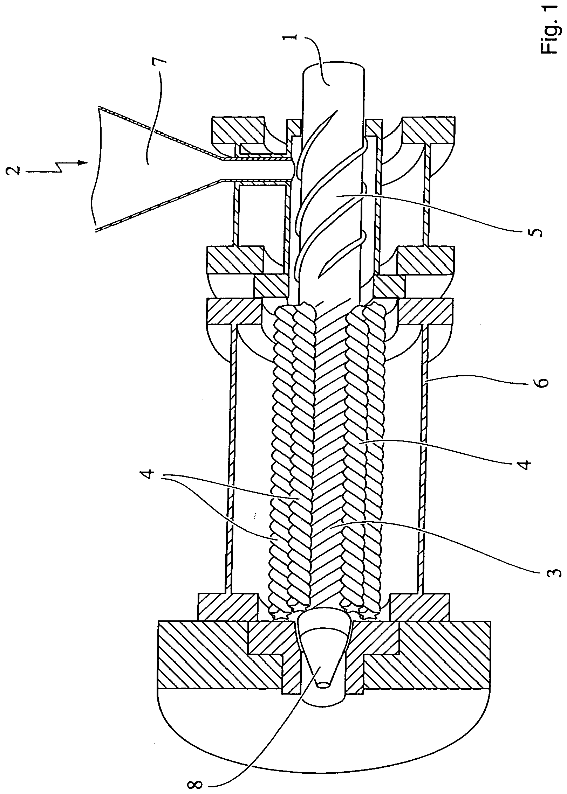

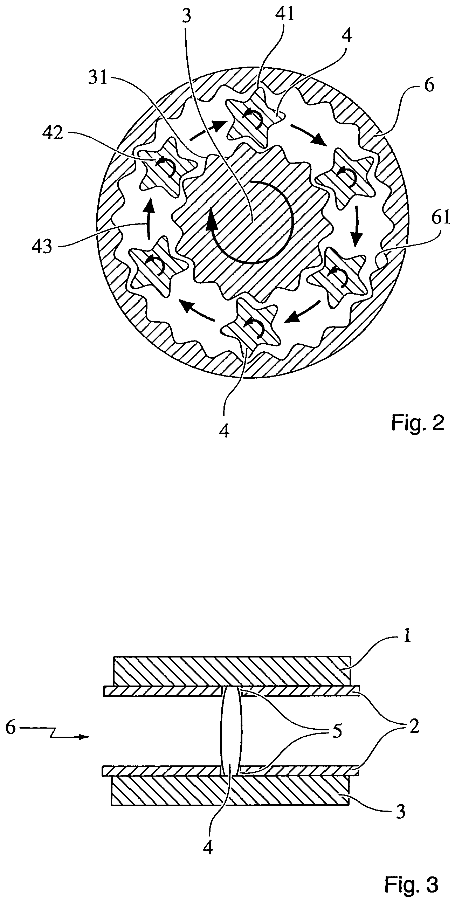

Im folgenden wird der Einsatz eines Planetwalzen-Extruders zur Herstellung der erfindungsgemäßen Darreichungsform anhand der Figuren 1 und 2 erläutert. Diese Erläuterungen sind lediglich beispielhaft und schränken den allgemeinen Erfindungsgedanken nicht ein.
- **Figur 1**: zeigt den Schnitt durch einen Planetwalzen-Extruder und
- **Figur 2**: zeigt die Wirkungsweise des Planetwalzen-Extruders.

In Figur 1 ist ein Planetwalzen-Extruder dargestellt, der vorzugsweise zur Herstellung der erfindungsgemäßen Darreichungsformen eingesetzt werden kann. Dieser Extruder weist im wesentlichen eine Welle 1 auf, die bezogen auf die Transportrichtung der zu extrudierenden Mischung der vorstehend aufgeführten Komponenten zunächst als Einzugsschnecke 5 und im weiteren als Zentralspindel 3 des Planetwalzen-Extruders gestaltet ist. Um die Zentralspindel 3 sind vorzugsweise drei bis sieben Planetspindeln 4 angeordnet, die wiederum von einem Mantel in Form eines Gehäuses 6 umgeben sind.

Im Planetwalzen-Extruder wird unter Bezugnahme auf Figur 1 die Extrusion der zum Einsatz kommenden Zusammensetzung zur Herstellung einer erfindungsgemäßen Darreichungsform vorzugsweise wie folgt durchgeführt. Wie durch Pfeil 2 dargestellt, werden die zu extrudierenden Komponenten durch die Dosiereinheit 7 in dem Bereich der Einzugsschnecke 5 dosiert und durch deren Drehung (Antrieb nicht dargestellt) in Richtung der Zentralspindel 3 befördert. Der Fachmann versteht, dass im Bereich der Einzugsschnecke ein Vermischen der Ausgangsstoffe (Komponenten) möglich ist. Es ist aber auch möglich, die Komponenten der Darreichungsform vorzumischen und diese Mischung über die Dosiereinheit 7 in dem Bereich der Einzugsschnecke 5 zu dosieren. Im Einzugsbereich des Planetwalzen-Extruders wird die Mischung gefördert. Durch Erwärmung bis mindestens zum Erweichungspunkt der Komponente (C) wird die Mischung aufgeschmolzen und dort im Bereich der Zentralspindel, d. h. im Extrusionsbereich die aufgeschmolzene Mischung durch Zusammenwirkung der Zentralspindel 3 und der Planetspindeln 4 gefördert, weiter homogenisiert, komprimiert bzw. kompaktiert und durch die Düsenbohrungen 8 als Extrusionsstränge extrudiert. Die Düsengeometrie bzw. die Geometrie der Bohrungen ist frei wählbar. So können die Bohrungen einen runden, oblongen oder ovalen Querschnitt aufweisen, wobei der runde Querschnitt vorzugsweise einen Durchmesser 0,1 mm bis 5 mm aufweist. Die Bohrungen weisen vorzugsweise einen runden Querschnitt auf. Sowohl der Mantel 6 des erfindungsgemäß zum Einsatz kommenden Planetwalzen-Extruders als auch die Zentralspindel können beheizt oder gekühlt werden. Die entsprechende Temperierung, d. h. Beheizung oder Kühlung, richtet sich danach, dass die zu extrudierende Mischung mindestens eine Durchschnittstemperatur entsprechend der Erweichungstemperatur der Komponente (C) aufweist und nicht über eine Temperatur steigt, bei der die zur verarbeitende Substanz (A) Schaden nehmen kann. Vorzugsweise wird die Temperatur der zu extrudierenden Mischung unter 180°C, vorzugsweise unter 150°C, aber mindestens auf die Erweichungstemperatur der Komponente (C), eingestellt. Die verwendeten Bezugszeichen beziehen sich ausschließlich auf Figuren 1 und 2.

Nach der Extrusion der geschmolzenen Mischung und gegebenenfalls Kühlung der extrudierten Stränge erfolgt eine nicht in Figur 1 dargestellte Vereinzelung der Extrudate. Diese Vereinzelung kann vorzugsweise durch Zerschneiden der Extrudate mittels mitlaufender oder rotierender Messer, Wasserstrahlschneider, Drähten, Klingen oder mit Hilfe von Laserschneidem durchgeführt werden.

Gegebenenfalls nach einem weiteren Abkühlen der vereinzelten Extrudate, die vorzugsweise in Scheiben vorliegen, erfolgt gegebenenfalls ein Umformen in die Endform der Darreichungsform, vorzugsweise durch Pelletisieren, wobei wenn nötig, wieder Wärmeeinwirkung erfolgt.

Die vereinzelten Extrudate, ggf. weiter verformt, werden vorzugsweise mit (restlichem) Sprengmittel (E) und ggf. Zusatzstoffen ausgerüstet.

So ausgerüstet können sie außer zu Tabletten verpresst, auch in multipartikulärer Form, wie als Pellets oder Spheriode, in Kapseln, Sachets, Stickpacks abgefüllt werden, um die erfindungsgemäße Darreichungsform als Dosiseinheit zu verwenden.

Figur 2 zeigt eine Querschnitt durch den Planetwalzen-Extruder. Um die sich drehende Zentralspindel 3 sind mindestens drei, in dem gezeigten Fall 6, Planetspindeln 4 angeordnet, deren Flanken 41 zu einem mit dem Flanken 31 der Zentralspindel 4 und zum anderen mit den Flanken 61 des Mantels 6 des Planetwalzen-Extruders zusammenwirken. Durch die Drehung der Zentralspindel 3 und das Abrollen der jeweiligen Flanken aufeinander drehen sich die Planetspindeln 4 jeweils wie mit Pfeil 42 um ihre eigene Achse und wie Pfeil 43 verdeutlicht, um die Zentralspindel 4 herum. Dadurch wird die angestrebte Komprimierung bzw. Kompaktierung der erfindungsgemäß zum Einsatz kommenden Komponentenmischung der erfindungsgemäß hergestellten Darreichungsformen bewirkt. Die verwendeten Bezugszeichen beziehen sich ausschließlich auf Figuren 1 und 2.

Sofern notwendig, kann der zum Einsatz kommende Planetwalzen-Extruder nicht nur einen Extrusionsbereich aufweisen, sondern mindestens noch einen weiteren, um gegebenenfalls auch die zu extrudierende Mischung entgasen zu können.

Das Verfahren kann diskontinuierlich oder kontinuierlich, vorzugsweise kontinuierlich, durchgeführt werden.

Als Extruder eignet sich beispielsweise ein Planetwalzen-Extruder mit vier Planetspindeln vom Typ BCG 10 der Fa. LBB Bohle (Ennigerloh, Deutschland) mit einer Extrusionsdüse mit einem Durchmesser von 8 mm. Eine gravimetrische Dosierung von 3,0 kg pro Stunde ist geeignet. Die Extrusion kann beispielsweise mit einer Drehzahl von 28,6 Upm bei einer Produkttemperatur von ca. 88 °C durchgeführt werden.

### Verfahrensvariante 4:

Zur Durchführung dieser Variante zur Herstellung der erfindungsgemäßen Darreichungsform werden wenigstens die Komponenten (A), (C), ggf. (D), ggf. zumindest ein Teil des Sprengmittels (E) und ggf. vorhandene Hilfsstoffe (B1), wie Antioxidantien und/oder Weichmacher unter Zugabe eines Lösungsmittels für die Komponente (C), d.h. für das oder die Polymere (C), zu der multipartikulären Darreichungsform verarbeitet.

Dazu werden die Komponenten (A), ggf. (B1), (C), die ggf. vorhandene Komponente (D) und ggf. zumindest ein Teil des Sprengmittels (E) gemischt und die resultierende Formulierungsmischung nach Zugabe des Lösungsmittels vereinzelt und ggf. weiter verformt.

Die Mischung der Komponenten kann in einem dem Fachmann bekannten Mischgerät erfolgen. Das Mischgerät kann beispielsweise ein Wälzmischer, Schüttelmischer, Schermischer oder Zwangsmischer sein.

Die Zugabe des Lösungsmittels für das Polymere (C) erfolgt zumindest in solchen Mengen, das die Formulierungsmischung gleichmäßig befeuchtet wird.

Als Lösungsmittel für das Polymere (C) eignen sich vorzugsweise wässrige Lösungsmittel, wie Wasser, Mischungen von Wasser und aliphatischen Alkoholen, vorzugsweise Alkoholen mit C1 bis C6, Estern, Ethern, Kohlenwasserstoffen, besonders bevorzugt destilliertes Wasser, kurzkettige Alkohole, wie Methanol, Ethanol, Isopropanol, Butanol oder wässrige Alkohollösungen.

Die Zugabe des Lösungsmittels erfolgt vorzugsweise unter Rühren. Anschließend wird die gleichmäßig befeuchtete Masse, vorzugsweise nach Vereinzelung getrocknet. Die Trocknung erfolgt vorzugsweise unter Wärmeeinwirkung bei Temperaturen, bei denen eine Verfärbung der Masse ausgeschlossen werden kann. Durch einfache Vorversuchte ist diese Temperatur feststellbar.

Es ist auch möglich, die Befeuchtung der Formulierungsmischung so durchzuführen, dass vor der Zugabe des Lösungsmittels die Formulierungsmischung, vorzugsweise in Formen auf Teilmassen verteilt, in einem flüssigen Dispergierungsmittel unter Rühren zu dispergieren und dann das Lösungsmittel zuzugeben. Die Komponente (C) ist in dem Dispergiermittel nicht löslich, das mit dem Lösungsmittel mischbar sein muss.

Als Dispergiermittel eignen sich vorzugsweise hydrophile Lösungsmittel, wie aliphatische Alkohole, Ketone, Ester. Kurzkettige Alkohole werden bevorzugt eingesetzt.

Alternativ kann die Befeuchtung der Formulierungsmischung auch so erfolgen, dass das Lösungsmittel als Schaum in die Formulierungsmischung eingearbeitet wird. Vorzugsweise wird ein solcher Schaum des Lösungsmittels mit Hilfe hochtouriger Mixer, vorzugsweise unter Zugabe üblicher Schaumstabilisatoren, hergestellt. Beispielsweise eignen sich als Stabilisatoren hydrophile Polymere wie z.B. Hydroxypropylmethylcellulose.

Vorzugsweise wird auch der Schaum unter Rühren in die Formulierungsmischung eingearbeitet, wodurch vorzugsweise eine granulierte Masse erhalten wird.

Die granulierte Masse wird getrocknet und anschließend zu der multipartikulären Darreichungsform geformt, z. B. durch Pelletisieren.

Die Trocknung und Formung kann vorzugsweise wie vorstehend angegeben erfolgen. Das erfindungsgemäße Verfahren kann auch so durchgeführt werden, dass zu der Formulierungsmischung soviel Lösungsmittel zugegeben wird, dass eine formbare Paste entsteht.

Eine solche Paste kann vor oder nach ihrer Trocknung, die wie vorstehend aufgeführt, durchgeführt werden kann, in Teilmassen aufgeteilt werden.

Diese Teilmassen können in Form von Strängen ausgebildet werden, die mit Hilfe eines Siebes oder eines Strangformers erzeugt werden können. Die getrockneten Stränge werden vorzugsweise vereinzelt und zur Darreichungsform endgeformt, z. B. durch Pelletisieren.

Es ist auch möglich, die Paste zu einem flächenförmigen Gebilde zu verarbeiten und aus dem getrockneten Gebilde die Darreichungsform zu stanzen.

Vorteilhafterweise wird die Paste mit Hilfe eines Extruders verarbeitet, wobei je nach Gestaltung der Extrusion diese Stränge oder flächenförmigen Gebilde erzeugt werden, die durch Abschlagen oder Schneiden bzw. Stanzen vereinzelt werden. Die vereinzelten Teilmassen können wie vorstehend ausgeführt zu der Darreichungsform endgeformt oder ausgestanzt werden. Entsprechende Vorrichtungen sind dem Fachmann bekannt.

Auf jeden Fall werden die endgeformten, multipartikulären Darreichungsformen noch ggf. mit der (restlichen) Menge der Komponente (E) und ggf. mit Zusatzstoffen (B2) versehen, bevor sie als Dosiseinheit abgefüllt oder verpresst werden. Das erfindungsgemäße Lösungs-Verfahren kann kontinuierlich oder diskontinuierlich durchgeführt werden.

Es ist auch möglich, zu der Formulierungsmischung soviel Lösungsmittel zuzugeben, dass zumindest die Polymerkomponente (C) gelöst wird. Eine solche Lösung oder Dispersion/Suspension wird vorzugsweise zu einem flächenförmigen Gebilde verarbeitet, wobei bevorzugt ein Extruder mit einer Flachdüse zum Einsatz kommt oder die Lösung auf eine flächenförmige, ebene Unterlage ausgegossen wird.

Nach Trocknung, wie vorstehend angegeben, können aus den flächenförmigen Gebilden die multipartikulären Darreichungsformen durch Stanzen oder Kalandrieren erhalten werden. Es ist auch möglich, die Lösung, wie vorstehend angegeben, zu Strängen zu verarbeiten und diese, bevorzugt nach ihrer Trocknung, zu vereinzeln und zur Darreichungsform zu formen.

Alternativ kann die Lösung auch in solchen Teilmengen aufgeteilt werden, dass sie nach dem Trocknen jeweils der Masse einer Einheit der Darreichungsform entspricht, wobei vorzugsweise dafür bereits Formen entsprechend der Form einer Einheit der Darreichungsform eingesetzt werden.

Sofern die Lösung in beliebige Teilmengen aufgeteilt wird, können die Teilmengen nach dem Trocknen gegebenenfalls wieder zu einer Dosiseinheit vereinigt werden, die z. B. in eine Kapsel abgefüllt oder zu einer Tablette verpresst werden kann.

Vorzugsweise werden die mit Lösungsmittel versetzten Formulierungsmischungen bei Temperaturen von 20°C bis 40°C verarbeitet, wobei außer bei der Trocknung zur Entfernung des Lösungsmittels und des ggf. vorhandenen Dispergierungsmittels keine höheren Temperaturen angewendet werden. Die Temperatur zum Trocknen muss unterhalb der Zersetzungstemperatur der Komponenten gewählt werden. Gegebenenfalls kann nach der Formgebung zur Darreichungsform nochmals eine Trocknung entsprechend der vorstehend beschriebenen Trocknung erfolgen.

Es sind auch Kombinationen einzelner Verfahrensschritte der vorstehenden Verfahrensvarianten möglich, um die erfindungsgemäße Darreichungsform herzustellen.

Die erfindungsgemäßen, multipartikulären Darreichungsformen werden bevorzugt mit einer Umhüllung aus Sprengmittel (E) umgeben, damit die IR-Freisetzung des Wirkstoffs gewährleistet wird. Zumindest ist es vorteilhaft, die Partikel, vorzugsweise Pellets, der erfindungsgemäßen Darreichungsform mit einem Sprengmittel (E) zu mischen und die Mischung vorzugsweise mit weiterem Füllstoff (B2), wie mikrokristalliner Cellulose, Magnesiumstearat, Calciumdhydrogenphosphat, Lactose, Fettsäureester, Mannitol, Hydroxypropylmethylcellulose, Pulvercellulose, Talkum, Siliciumdioxid, Collidon, Zuckerester und/oder Polyvinylpyrrolidon zu verdünnen. Solche Mischungen können als Dosiseinheit in Kapseln, Sachets oder Stickpacks abgefüllt oder zu Kautabletten, dispergierbaren Tabletten oder IR-Tabletten verarbeitet werden. Ganz besonders bevorzugt weisen die Partikel, Pellets der erfindungsgemäßen Darreichungsform einen Überzug umfassend mindestens ein Sprengmittel (E) auf, der durch Pulverbeschichtung oder Filmbeschichtung aufgebracht wurde. Die aufgeführten Dosiseinheiten können ja nach Anwendungszweck außerdem Aromastoffe, Brausezusätze, Zucker, Süßstoffe und/oder Farbstoffe aufweisen.

Wenn die erfindungsgemäße Darreichungsform die Komponente (c) und/oder (e) enthält, ist die Dosierung so zu wählen, dass bei bestimmungsgemäßer oraler Applikation keine negative Wirkung hervorgerufen wird. Wird jedoch die vorgesehene Dosierung bei einem Missbrauch überschritten, wird Übelkeit bzw. Brechreiz bzw. schlechter Geschmack hervorgerufen. Die jeweilige Menge der Komponente (c) und/oder (e), die vom Patienten bei bestimmungsgemäßer oraler Applikation noch toleriert wird, kann vom Fachmann durch einfache Vorversuche ermittelt werden.

Sofern aber unabhängig von der praktisch nicht möglichen Pulverisierbarkeit der erfindungsgemäßen Darreichungsform zur Sicherung der Darreichungsform der Einsatz der Komponenten (b) und/oder (c) und/oder (e) vorgesehen ist, sollten diese Komponenten bevorzugt in einer so hohen Dosierung zum Einsatz kommen, dass sie bei einer missbräuchlichen Applikation der Darreichungsform eine intensive negative Wirkung beim Missbraucher hervorrufen. Dies gelingt vorzugsweise durch eine räumliche Trennung zumindest des Wirkstoffes bzw. der Wirkstoffe von den Komponenten (b) und/oder (c) und/oder (e), wobei bevorzugt der Wirkstoff bzw. die Wirkstoffe in wenigstens einer Untereinheit (X) und die Komponenten (b) und/oder (c) und/oder (e) in wenigstens einer Untereinheit (Y) vorliegen, und wobei die Komponenten (b), (e) bei bestimmungsgemäßer Applikation der Darreichungsform bei Einnahme und/oder im Körper nicht ihre Wirkung entfalten und die übrigen Formulierungskomponenten insbesondere die Komponente (C) und ggf. (D) und (E) identisch sind.

Sofern die erfindungsgemäße Darreichungsform wenigstens 2 der Komponenten (b) und (c) bzw. (e) aufweist, können diese jeweils in derselben oder in verschiedenen Untereinheiten (Y) vorliegen. Vorzugsweise liegen, sofern vorhanden, alle Komponenten (b), (c) und (e) in ein- und derselben Untereinheit (Y) vor. Untereinheiten im Sinne der vorliegenden Erfindung sind feste Formulierungen, die jeweils neben üblichen, dem Fachmann bekannten Hilfsstoffen den (die) Wirkstoff(e), mindestens ein Polymer (C) und wenistens ein Sprengmittel (E) die gegebenenfalls vorhandene Komponente (D) und gegebenenfalls wenigstens eine der gegebenenfalls vorhandenen Komponenten (a) und/oder (e) bzw. jeweils wenigstens ein Polymer (C) und gegebenenfalls (D) und wenigstens ein Sprengmittel (E) und den (die) Antagonist(en) und/oder das Emetikum (die Emetika) und/oder die Komponente (d) und/oder die Komponente (e) und gegebenenfalls wenigstens eine der gegebenenfalls vorhandenen Komponenten (a) enthalten. Dabei ist darauf zu achten, dass jede der genannten Untereinheiten nach den vorstehend angegebenen Verfahren formuliert werden.

Ein wesentlicher Vorteil der getrennten Formulierung der Wirkstoffe von den Komponenten (b) bzw. (c) bzw. (e) in Untereinheiten (X) und (Y) der erfindungsgemäßen Darreichungsform besteht darin, dass bei ihrer bestimmungsgemäßen Applikation die Komponenten (b) und/oder (c) und/oder (e) bei Einnahme und/oder im Körper praktisch nicht freigesetzt werden oder nur in so geringen Mengen freigesetzt werden, dass sie keine den Patienten oder den Therapieerfolg beeinträchtigende Wirkung entfalten oder bei der Passage durch den Körper des Patienten nur an solchen Freisetzungsorten abgegeben werden, an denen eine für ihre Wirksamkeit ausreichende Resorption nicht gegeben ist. Vorzugsweise werden die Komponenten (b) und/oder (c) und/oder (e) bei bestimmungsgemäßer Applikation der Darreichungsform im Körper des Patienten praktisch nicht freigesetzt oder vom Patienten nicht wahrgenommen.

Der Fachmann versteht, dass diese vorstehend genannten Bedingungen in Abhängigkeit von den jeweils eingesetzten Komponenten (b), (c) und/oder (e) sowie der Formulierung der Untereinheiten bzw. der Darreichungsform variieren können. Die für die jeweilige Darreichungsform optimale Formulierung kann durch einfache Vorversuche ermittelt werden. Entscheidend ist, dass die jeweiligen Untereinheiten das Polymer (C), das Sprengmittel (E) und gegebenenfalls die Komponente (D) enthalten und in der vorstehend angegebenen Weise formuliert wurden.

Sollte es den Missbrauchern wider Erwarten gelingen, eine solche erfindungsgemäße Darreichungsform, welche die Komponenten (b), (c) und/oder (e) in Untereinheiten (Y) aufweist, zum Zwecke der mißbräuchlichen Einnahme des Wirkstoffes zu zerkleinern und ein Pulver zu erhalten, das mit einem geeigneten Extraktionsmittel extrahiert wird, wird neben dem Wirkstoff auch die jeweilige Komponente (b), (c) und/oder (e) in einer Form erhalten, in der sie von dem Wirkstoff nicht auf einfache Weise zu separieren ist, so dass sie bei der Applikation der manipulierten Darreichungsform, insbesondere bei oraler und/oder parenteraler Verabreichung, ihre Wirkung bei Einnahme und/oder im Körper entfaltet und zusätzlich eine der Komponente (b) und/oder (c) und/oder (e) entsprechende negative Wirkung beim Missbraucher hervorruft oder ein Versuch, den Wirkstoff zu extrahieren durch die Farbgebung abschreckt und so den Missbrauch der Darreichungsform verhindert.

Die Formulierung einer erfindungsgemäßen Darreichungsform, in der eine räumliche Trennung des Wirkstoffes bzw. der Wirkstoffe von den Komponenten (b), (c) und/oder (e), vorzugsweise durch Formulierung in verschiedenen Untereinheiten erfolgt ist, kann in vielfältiger Art und Weise erfolgen, wobei die entsprechenden Untereinheiten in der erfindungsgemäßen Darreichungsform jeweils in beliebiger räumlicher Anordnung zueinander vorliegen können, sofern die vorstehend genannten Bedingungen für die Freisetzung der Komponenten (b) und/oder (c) und/oder (e) erfüllt sind.

Der Fachmann versteht, dass die ggf. auch vorliegenden Komponente(n) (a) bevorzugt sowohl in den jeweiligen Untereinheiten (X) und (Y) als auch in Form von eigenständigen, den Untereinheiten (X) und (Y) entsprechenden Untereinheiten in der erfindungsgemäßen Darreichungsform formuliert werden können, so lange die Sicherung der Darreichungsform gegen den Missbrauch wie auch die Wirkstofffreisetzung bei bestimmungsgemäßer Applikation durch die Art der Formulierung nicht beeinträchtig werden und das Polymer (C), das Sprengmittel (E) und gegebenenfalls (D) mit formuliert und die Formulierung gemäß den vorstehend angegebenen Verfahren zur Erzielung der notwendigen Härte durchgeführt wird.

In einer bevorzugten Ausführungsform der erfindungsgemäßen Darreichungsform liegen die beiden Untereinheiten (X) und (Y) in multipartikulärer Form vor, wobei Mikrotabletten, Granulaten, Sphäroiden, Perlen oder Pellets bevorzugt sind und sowohl für die Untereinheit (X) als auch (Y) dieselbe Form, d.h. Gestaltung gewählt wird, damit keine Separierung der Untereinheiten (X) von (Y), z. B. durch mechanische Auslese, möglich ist. Die multipartikulären Formen weisen bevorzugt eine Größe im Bereich von 0,1 bis 5 mm, vorzugsweise 0,2 bis 3 mm auf.

Die erfindungsgemäße Darreichungsform liegt in multipartikulärer Form, bevorzugt in Form von Mikrotabletten, Granulaten, Sphäroiden, Perlen oder Pellets, ggf. als Dosiseinheit in Kapseln abgefüllt oder zu Tabletten verpreßt, zur oralen Verabreichung vor. Vorzugsweise weisen die multipartikulären Formen eine Größe im Bereich von 0,1 bis 5 mm, besonders bevorzugt im Bereich von 0,2 bis 3 mm auf (Bestimmungsmethode gemäß veröffentlichter Dissertation "Systematische Untersuchungen zur Eignung von kappa-Carrageenan als Pelletierhilfsstoff in der Feuchtextrusion/Sphäronisation, Seiten 16, 21-23 von Markus Thommes in der "Deutschen Bibliothek" in der Deutschen Nationalbibliographie, 1. Auflage Cuvillin Verlag, Göttingen, 2006)

Die Untereinheiten (X) und (Y) in multpartikulärer Form können auch bevorzugt in eine Kapsel, Sachets, Stickpacks abgefüllt oder zu einer Tablette verpresst werden, wobei die Untereinheiten (X) und (Y) auch in der resultierenden Dosiereinheiten erhalten bleiben.

Die jeweiligen multipartikulären Untereinheiten (X) bzw. (Y) mit identischer Formgebung sollten auch nicht visuell voneinander unterscheidbar sein, damit sie vom Missbraucher nicht durch einfaches Sortieren voneinander separiert werden können.

Vorzugsweise wird die Freisetzung der Komponente (b), (c) und/oder (e) aus der Untereinheit (Y) der erfindungsgemäßen Darreichungsform mit Hilfe einer Umhüllung verhindert, so dass die Untereinheit aus üblichen, dem Fachmann bekannten Materialien bestehen kann, sofern sie wenigstens ein Polymer (C), und gegebenenfalls (D) zur Erfüllung der Härtebedingung der erfindungsgemäßen Darreichungsform enthält und mit Sprengmittel (E) versehen ist.

Bevorzugt können für eine Umhüllung die nachstehend aufgeführten Materialien zum Einsatz kommen.

Bevorzugte Materialien sind solche, die ausgewählt sind aus der Gruppe umfassend Alkylcellulosen, Hydroxyalkylcellulosen, Glucanen, Skleroglucanen, Mannanen, Xanthanen, Copolymeren aus Poly[bis(p-carboxyphenoxy)propan und Sebacinsäure, vorzugsweise in einem Molverhältnis von 20:80 (unter der Bezeichnung Polifeprosan 20^{®} am Markt geführt), Carboxymethylcellulosen, Celluloseethern, Celluloseestern, Nitrocellulosen, Polymeren auf Basis von (Meth)acrylsäure sowie deren Estern, Polyamiden, Polycarbonaten, Polyalkylenen, Polyalkylenglykolen, Polyalkylenoxiden, Polyalkylenterephtalate, Polyvinylalkohole, Polyvinylether, Polyvinylester, halogenierte Polyvinyle, Polyglykolide, Polysiloxane, Polyurethane, deren Copolymeren und deren Mischungen.

Besonders geeignete Materialien können ausgewählt werden aus der Gruppe umfassend Methylcellulose, Ethylcellulose, Hydroxypropylcellulose, Hydroxypropylmethylcellulose, Hydroxybutylmethylcellulose, Celluloseacetat, Cellulosepropionat (von niederem, mittlerem oder erhöhtem Molekulargewicht), Celluloseacetatpropionat, Celluloseacetatbutyrat, Celluloseacetatphtalat, Carboxymethylcellulose, Cellulosetriacetat, Natrium-Cellulosesulfat, Polymethylmethacrylat, Polyethylmethacrylat, Polybutylmethacrylat, Polyisobutylmethacrylat, Polyhexylmethacrylat, Polyisodecylmethacrylat, Polylaurylmethacrylat, Polyphenylmethacrylat, Polymethylacrylat, Polyisopropylacrylat, Polyisobutylacrylat, Polyoctatdecylacrylat, Polyethylen, Polyethylen niederer Dichte, Polyethylen hoher Dichte, Polypropylen, Polyethylenglykol, Polyethylenoxid, Polyethylenterephtalat, Polyvinylalkohol, Polyvinylisobutylether, Polyvinylacetat und Polyvinylchlorid.

Besonders geeignete Copolymere können ausgewählt werden aus der Gruppe umfassend Copolymere aus Butylmethacrylat und Isobutylmethacrylat, Copolymere aus Methylvinylether und Maleinsäure mit erhöhtem Molekulargewicht, Copolymere aus Methylvinylether und Maleinsäuremonoethylester, Copolymere aus Methylvinylether und Maleinsäureanhydrid sowie Copolymere aus Vinylalkohol und Vinylacetat.

Weitere, zur Formulierung einer Umhüllung besonders geeignete Materialien sind Stärke gefülltes Polycaprolacton (WO98/20073), aliphatische Polyesteramide (DE 19 753 534 A1, DE 19 800 698 A1, EP 0 820 698 A1), aliphatische und aromatische Polyesterurethane (DE 19822979), Polyhydroxyalkanoate, insbesondere Polyhydroxybutyrate, Polyhydroxyvaleriate), Casein (DE 4 309 528), Polylactide und Copolylactide (EP 0 980 894 A1).

Ggf. können die vorstehend genannten Materialien mit weiteren üblichen, dem Fachmann bekannten Hilfsstoffen, vorzugsweise ausgewählt aus der Gruppe umfassend Weichmacher, Gleitmittel, Antioxidantien, wie z. B. Glycerinmonostearat, halbsynthetische Triglyceridderivate, halbsynthetische Glyceride, hydriertes Rizinusöl, Glycerinpalmitostearat, Glycerinbehenat, Polyvinylpyrrolidon, Gelatine, Magnesiumstearat, Stearinsäure, Natriumstearat, Talkum, Natriumbenzoat, Borsäure und kolloidalem Silica, Fettsäuren, substituierte Triglyceride, Glyceride, Polyoxyalkylenglykole, Polyalkylenglykole und deren Derivate abgemischt werden.

Die erfindungsgemäße Darreichungsform weist eine IR-Freisetzung, wie vorstehend definiert, des Wirkstoffes auf. Sie eignet sich daher vorzugsweise für eine Behandlung, bei der eine schnell einsetzende Wirkung erzielt werden soll, wie z. B. für eine akute Schmerzbekämpfung.

### Methode zur Bestimmung der Bruchfestigkeit

Zur Überprüfung, ob ein Material als Komponente (C) oder (D) eingesetzt werden kann, wird das Material zu einer Tablette mit einem Durchmesser von 10 mm und einer Höhe von 5mm mit einer Kraft von 150 N, bei einer Temperatur entsprechend mindestens dem Erweichungspunkt des Materials und bestimmt mit Hilfe eines DSC-Diagramms des Materials verpresst. Mit so hergestellten Tabletten wird gemäß der Methode zur Bestimmung der Bruchfestigkeit von Tabletten, veröffentlicht im Europäischen Arzneibuch 1997, Seite 143, 144, Methode Nr. 2.9.8. unter Einsatz der nachstehend aufgeführten Apparatur die Bruchfestigkeit bestimmt. Als Apparatur für die Messung wird eine Zwick Materialprüfmaschine "Zwick Z 2.5", Materialprüfmaschine Fmax 2.5 kN mit einem Traversenweg von max. 1150 mm, der durch einen Aufbau mit Hilfe einer Säule und einer Spindel einzustellen ist, einen freien Arbeitsraum nach hinten von 100 mm und einer zwischen 0,1 bis 800 mm /min. einstellbaren Prüfgeschwindigkeit und einer Software: testControl eingesetzt. Es wird ein Druckstempel mit schraubbaren Einsätzen und einem Zylinder (Durchmesser 10 mm), ein Kraftaufnehmer, Fmax. 1 kN, Durchmesser 8 mm, Klasse 0.5 ab 10 N, Klasse 1 ab 2 N nach ISO 7500-1, mit Hersteller-Prüfzertifikat M nach DIN 55350-18 (Zwick-Bruttokraft Fmax 1,45 kN) zur Messung eingesetzt (alles Apparaturen der Firma Zwick GmbH & Co. KG, Ulm, Deutschland) mit der Bestell-Nr. BTC-FR 2.5 TH. D09 für die Prüfmaschine, der Bestell-Nr. BTC-LC 0050N. P01 für den Kraftaufnehmer, der Bestell-Nr. BO 70000 S06 für die Zentriervorrichtung.

**Figur 3** zeigt die Messung der Bruchfestigkeit einer Tablette, insbesondere die dafür eingesetzte Justierungsvorrichtung (6) der Tablette (4) vor und während der Messung. Darzu wird die Tablette (4) zwischen der oberen Druckplatte (1) und der unteren Druckplatte (3) der nicht dargestellten Vorrichtung zur Kraftaufbringung mit Hilfe von zwei 2-teiligen Einspannvorrichtungen, die jeweils mit der oberen bzw. unteren Druckplatte nach Einstellung des zur Aufnahme und zur Zentrierung der zu messenden Tablette notwendigen Abstands (5) fest verbunden (nicht dargestellt) werden. Zur Einstellung des Abstands (5) können die 2-teiligen Einspannvorrichtungen jeweils auf der Druckplatte, auf der sie gelagert sind, horizontal nach außen oder innen bewegt werden.

Als bruchfest bei einer bestimmten Krafteinwirkung werden auch die Tabletten eingestuft, bei denen kein Bruch feststellbar , aber ggf. eine plastische Verformung ohne Zerteilung der Tablette durch die Krafteinwirkung erfolgt ist.

Im Folgenden wird die Erfindung anhand von Beispielen erläutert. Diese Erläuterungen sind lediglich beispielhaft und schränken den allgemeinen Erfindungsgedanken nicht ein.

### Beispiele:

In einer Reihe von Beispielen wurde Tramadolhydrochlorid als Wirkstoff verwendet. Tramadolhydrochlorid wurde eingesetzt, obwohl Tramadol kein Wirkstoff mit üblichem Mißbrauchspotential ist und daher nicht unter das Betäubungsmittelgesetz fällt, um das experimentelle Arbeiten zu erleichtern. Tramadol ist außerdem ein Vertreter der Klasse der Opioide mit ausgezeichneter Wasserlöslichkeit.

### Beispiel 1

### 1.1. Pelletherstellung

### Zusammensetzung der Pellets:

| | | Anteil [%] |
|---|---|---|
| Tramadol HCl | 50 mg | 40% |
| Polyethylenoxid, NF 7 000 000 (MG) | 50 mg | 40% |
| (Polyox WSR 303, Dow Chemicals) | | |
| (Metholose 90 SH, 100000 cP) Hydroxypropylmethylcellulose (Shin-Etsu) | 12,5 mg | 10% |
| PEG 6000 (Polyethylenglykol) | 12,5 mg | 10% |

Die Komponenten wurden eingewogen und in einem Freifallmischer für 15 Minuten gemischt. Anschließend wurden sie mit einem Doppelschneckenextruder der Fa. Leistritz Typ ZSE18HP40D mit Micropelletizer extrudiert. Es wurden exzentrische Schneckenspitzen und ein Verdrängerkegel verwendet. Die Düsenplatte hatte acht Bohrungen mit einem Durchmesser von 1,0 mm und das Verhältnis Länge/Durchmesser betrug 2,5. Die entstandenen Pellets hatten eine Länge von 1 mm ± 20%. Die Prozessparameter waren folgende:

| | |
|---|---|
| Zylindertemperatur HZ1 Soll 40°C /Ist: | 39,6°C |
| Zylindertemperatur HZ2 und HZ2 | 100°C |
| Zylindertemperatur HZ4 bis HZ8 | 120°C |
| Zylindertemperatur HZ10 | 120°C |
| Zylindertemperatur HZ11 | 140°C |
| Produkttemperatur am Austragsteil | 134,1 °C |
| Ausstoß | 33,43 g/min |
| Schneckendrehzahl (1/min) | 150/min |

Die Bruchfestigkeit der Pellets wurde nach der vorstehend beschriebenen Methode mit der in Figur 3 dargestellten Apparatur bestimmt. Bei der Krafteinwirkung von 500 N trat kein Bruch auf. Die Pellets konnten mit einem Hammer nicht zerkleinert werden, auch mit Hilfe von Mörser und Pistill war dies nicht möglich.

### 1.2. Kapselherstellung

### Zusammensetzung der Kapselfüllung

| | | |
|---|---|---|
| Pellets nach Beispiel 1.1. hergestellt (≅ 50 mg Tramadol) | 125 mg | 95% |
| Crosspovidone | 6,3 mg | 5% |
| Gesamtmenge | 131,3 mg | 100% |

Die nach 1.1. hergestellten Pellets wurden mit Crosspovidone solange gemischt, bis eine homogene Mischung erhalten wurde. Diese Mischung wurde in Gelatine-Steckkapseln der Größe 0 gefüllt.

Die in vitro Freisetzung von Tramadol aus der Kapsel wurde in einer Blattrührerapparatur mit Sinker nach Pharm Eur. Bestimmt. Die Temperatur des Freisetzungsmediums betrug 37°C und die Umdrehungsgeschwindigkeit des Rührers 75 min⁻¹. Als Freisetzungsmedium wurden 600 ml Puffer pH 1,2 verwendet. Die jeweils zu einem Zeitpunkt im Lösungsmedium freigesetzte Menge an Tramadol wurde spektralphotometrisch (bei 271 nm) bestimmt.

| Zeit | Freigesetzte Wirkstoffmenge |
|---|---|
| 15 min | 56% |
| 30 min | 80% |
| 45 min | 89% |

### Beispiel 2

### 2.1. Pellets, die gemäß Beispiel 1.1. hergestellt wurden, werden verwendet.

### 2.2. Zusammensetzung der Kapselfüllung

| | | |
|---|---|---|
| Pellets nach 1.1. hergestellt (≅ 50 mg Tramadol) | 125 mg | 95% |
| Crosscarmellose | 6,3 mg | 5% |
| Gesamtmenge | 131,3 mg | 100% |

Die Pellets wurden mit Crosscarmellose gemischt und in Gelatine-Steckkapseln der Größe 0 gefüllt.

Die in vitro Freisetzung des Tramadols aus der Kapsel wurde in der Blattrührerapparatur mit Sinker nach Pharm Eur. bestimmt. Die Temperatur des Freisetzungsmediums betrug 37°C und die Umdrehungsgeschwindigkeit des Rühers 75 min⁻¹. Als Freisetzungsmedium wurden 600 ml Puffer pH 1,2 verwendet. Die jeweils zu einem Zeitpunkt im Lösungsmedium freigesetzte Menge an Wirkstoff wurde spektralphotometrisch (bei 271 nm) bestimmt.

| Zeit | Freigesetzte Wirkstoffmenge |
|---|---|
| 15 min | 44% |
| 30 min | 71% |
| 45 min | 82% |

### Beispiel 3

### 3.1. Es werden Pellets, die gemäß Beispiel 1.1. hergestellt wurden, verwendet.

### 3.2. Zusammensetzung der Kapselfüllung

| | | |
|---|---|---|
| Pellets nach Beispiel 1.1. hergestellt (≅ 50 mg Tramadol) | 125 mg | 73,8% |
| Crosspovidone | 6,3 mg | 3,7% |
| Mikrokristalline Cellulose (Avicel PH101) | 37,5 mg | 22,1% |
| Magnesiumstearat | 0,6 mg | 0,4% |
| Gesamtmenge | 169,4 mg | 100% |

Die Pellets wurden mit Crosscarmellose, mikrokristalliner Cellulose und Magnesiumstearat gemischt und in Gelatine-Steckkapseln der Größe 0 abgefüllt.

Die in vitro Freisetzung des Tramadols aus der Kapsel wurde in der Blattrührerapparatur mit Sinker nach Pharm Eur. bestimmt. Die Temperatur des Freisetzungsmediums betrug 37°C und die Umdrehungsgeschwindigkeit des Rühers 75 min⁻¹. Als Freisetzungsmedium wurden 600 ml Puffer pH 1,2 verwendet. Die jeweils zu einem Zeitpunkt im Lösungsmedium freigesetzte Menge an Wirkstoff wurde spektralphotometrisch (bei 271 nm) bestimmt.

| Zeit | Freigesetzte Wirkstoffmenge |
|---|---|
| 15 min | 70% |
| 30 min | 88% |
| 45 min | 92% |

### Beispiel 4

### 4.1. Es wurden Pellets, die gemäß Beispiel 1.1. hergestellt wurden, verwendet.

### 4.2. Zusammensetzung der Kapselfüllung

| | | |
|---|---|---|
| Pellets nach Beispiel 1.1. hergestellt (≅ 50 mg Tramadol) | 125 mg | 66,7% |
| Crosscarmellose | 6,25 mg | 3,3% |
| Mikrokristalline Cellulose (Avicel PH101) | 50,0 mg | 26,7% |
| Calciumdihydrogenphosphat | 6,25 mg | 3,3% |
| Gesamtmenge | 187,5 mg | 100% |

Die Pellets wurden mit den Hilfsstoffen gemischt und in Gelatine-Steckkapseln der Größe 0 abgefüllt.

Die in vitro Freisetzung des Tramadols aus der Kapsel wurde in der Blattrührerapparatur mit Sinker nach Pharm Eur. bestimmt. Die Temperatur des Freisetzungsmediums betrug 37°C und die Umdrehungsgeschwindigkeit des Rühers 75 min⁻¹. Als Freisetzungsmedium wurden 600 ml Puffer pH 1,2 verwendet. Die jeweils zu einem Zeitpunkt im Lösungsmedium freigesetzte Menge an Wirkstoff wurde spektralphotometrisch (bei 271 nm) bestimmt.

| Zeit | Freigesetzte Wirkstoffmenge |
|---|---|
| 15 min | 51% |
| 30 min | 80% |
| 45 min | 88% |

### Beispiel 5

### 5.1. Es wurden die Pellets, die gemäß Beispiel 1.1. hergestellt wurden, verwendet.

### 5.2. Zusammensetzung der Kapselfüllung

| | | |
|---|---|---|
| Pellets nach Beispiel 1.1. hergestellt (≅ 50 mg Tramadol) | 125 mg | 95,2% |
| Crosspovidone mikronisiert | 6,25 mg | 4,8 % |
| Gesamtmenge | 131,25 mg | 100% |

Crosspovidone (mikronisiert) und Pellets wurden in einem high sheer mixer (Diosna Laborgranulator 4) 15 Minuten lang gemischt. Die beschichteten Pellets wurden in eine Gelatine-Kapsel der Größe 0 abgefüllt.

Die Bruchfestigkeit der Pellets wurde nach der Methode, wie vorstehend beschrieben, mit der dort angegebenen Apparatur bestimmt. Bei einer Kraftreinwirkung von 500 N trat kein Bruch auf. Die Pellets konnten mit einem Hammer nicht zerkleinert werden. Auch mit Hilfe von Mörser und Pistill war dies nicht möglich.

Die in vitro Freisetzung des Tramadols aus der Kapsel wurde in der Blattrührerapparatur mit Sinker nach Pharm Eur. bestimmt. Die Temperatur des Freisetzungsmediums betrug 37°C und die Umdrehungsgeschwindigkeit des Rühers 75 min⁻¹. Als Freisetzungsmedium wurden 600 ml Puffer pH 1,2 verwendet. Die jeweils zu einem Zeitpunkt im Lösungsmedium freigesetzte Menge an Wirkstoff wurde spektralphotometrisch (bei 271 nm) bestimmt.

| Zeit | Freigesetzte Wirkstoffmenge |
|---|---|
| 15 min | 52% |
| 30 min | 77% |
| 45 min | 86% |

### Beispiel 6

### 6.1. Pelletherstellung

### Zusammensetzung der Pellets

| | Pro Kapselfüllung | Anteil [%] |
|---|---|---|
| Tramadol HCl | 50 mg | 45% |
| Polyethylenoxid 7 000 000 (MG) (Polyox WSR 303, Dow Chemicals) | 44,4 mg | 40% |
| Macrogol 6000 (Polyethylenglykol 6000 BASF) | 11,1 mg | 10% |
| Crosspovidone | 5,6 mg | 5% |

Die Komponenten wurden eingewogen und in einem Freifallmischer 15 Minuten lang gemischt. Anschließend wurden sie mit Hilfe eines Doppelschneckenextruder der Fa. Leistritz Typ ZSE18HP40D mit Micropelletizer extrudiert. Es wurden exzentrische Schneckenspitzen und ein Verdrängerkegel verwendet. Die Düsenplatte hatte acht Bohrungen mit einem Durchmesser von 1,0 mm, wobei das Verhältnis Länge/Durchmesser 2,5 betrug. Die Pellets hatten eine Länge von 1 mm ± 20%. Die Extrusionsparameter waren folgende:

| | |
|---|---|
| Zylindertemperatur HZ1 Soll 40°C /Ist: | 39,6°C |
| Zylindertemperatur HZ2 und HZ2 | 100°C |
| Zylindertemperatur HZ4 bis HZ8 | 120°C |
| Zylindertemperatur HZ10 | 120°C |
| Zylindertemperatur HZ11 | 140°C |
| Produkttemperatur am Austragsteil | 134,1 °C |
| Ausstoß | 33,43 g/min |
| Schneckendrehzahl (1/min) | 150/min |

Die Bruchfestigkeit der Pellets wurde nach der vorstehend beschriebenen Methode mit der dort angegebenen Apparatur bestimmt. Bei einer Krafteinwirkung von 500 N trat kein Bruch auf. Die Pellets konnten mit einem Hammer nicht zerkleinert werden; auch mit Hilfe von Mörser und Pistill war dies nicht möglich.

### 6.2. Zusammensetzung der Kapselfüllung

| | | |
|---|---|---|
| Pellets nach Beispiel 6.1. hergestellt (≅ 50 mg Tramadol) | 111 mg | 100% |
| Gesamtmenge | 111 mg | 100% |

Die nach 6.1. hergestellten Pellets wurden in Gelatine-Steckkapseln der Größe 0 abgefüllt.

Die in vitro Freisetzung des Tramadols aus der Kapsel wurde in der Blattrührerapparatur mit Sinker nach Pharm Eur. bestimmt. Die Temperatur des Freisetzungsmediums betrug 37°C und die Umdrehungsgeschwindigkeit des Rühers 75 min⁻¹. Als Freisetzungsmedium wurden 600 ml Puffer pH 1,2 verwendet. Die jeweils zu einem Zeitpunkt im Lösungsmedium freigesetzte Menge an Wirkstoff wurde spektralphotometrisch (bei 271 nm) bestimmt.

| Zeit | Freigesetzte Wirkstoffmenge |
|---|---|
| 15 min | 55% |
| 30 min | 77% |
| 45 min | 87% |

## Patentansprüche

1. Multipartikuläre Darreichungsform mit erschwertem Missbrauch umfassend
- wenigstens einen Wirkstoff mit Missbrauchspotential (A), welche eine psychotrope Wirkung aufweist, ausgewählt aus der Gruppe der Opioide;
- wenigstens ein synthetisches oder natürliches Polymer (C):
- ggf. wenigstens ein natürliches, halbsynthetisches oder synthetisches Wachs (D);
- wenigstens ein Sprengmittel (E), welches zumindest teilweise mit den Partikeln der Darreichungsform vermischt ist;
- einen Zusatzhilfsstoff (B2), der nicht Bestandteil der Partikel ist, ausgewählt aus der Gruppe der Füllstoffe; und
- ggf. einen oder mehrere, weitere physiologisch verträgliche Hilfsstoffe (B),
wobei die einzelnen Partikel der Darreichungsform eine Bruchfestigkeit von mindestens 500 N gemessen nach Pharm. Eur. und eine Wirkstoff-Freisetzung von wenigstens 75% nach 45 Minuten gemessen nach Pharm. Eur. in der Blattrührapparatur mit Sinker in 600 ml wässriger Pufferlösung mit einem pH-Wert von 1,2 bei 37 °C und 75 Umdrehungen pro min aufweisen.

2. Darreichungsform nach Anspruch 1, **dadurch gekennzeichnet, dass** sie in Form von Mikrotabletten, Mikropellets, Granulaten, Spheroiden, Perlen oder Pellets vorliegt.

3. Darreichungsform nach einem der Ansprüche 1 oder 2, **dadurch gekennzeichnet, dass** als Polymer (C) wenigstens ein Polymer ausgewählt aus der Gruppe umfassend Polyalkylenoxide, Polyethylene, Polypropylene, Polyvinylchloride, Polycarbonate, Polystyrole, Polyacrylate und deren Copolymerisate vorliegt.

4. Darreichungsform nach einem der vorstehenden Ansprüche, **dadurch gekennzeichnet, dass** als Polymer (C) wenigstens ein Polyalkylenoxid ausgewählt aus der Gruppe umfassend Polymethylenoxide, Polyethylenoxide, Polypropylenoxide, deren Copolymerisate, deren Blockcopolymerisate und deren Mischungen, vorzugsweise wenigstens ein Polyethylenoxid vorliegt.

5. Darreichungsform nach Anspruch 3 oder 4, **dadurch gekennzeichnet, dass** das Polyalkylenoxid ein Molekulargewicht von mindestens 0,5×10⁶ basierend auf rheologischen Messungen aufweist.

6. Darreichungsform nach Anspruch 5, **dadurch gekennzeichnet, dass** das Polyalkylenoxid ein Molekulargewicht von mindestens 1×10⁶ basierend auf rheologischen Messungen aufweist.

7. Darreichungsform nach einem der vorstehenden Ansprüche, **dadurch gekennzeichnet, dass** sie 0,1 bis 15 Gew.-%, vorzugsweise 1 bis 10 Gew.-%, besonders bevorzugt 3 bis 7 Gew.-%, bezogen auf das Gesamtgewicht der Darreichungsform, wenigstens eines Sprengmittels (E) aufweist.

8. Darreichungsform nach einem der vorstehenden Ansprüche, **dadurch gekennzeichnet, dass** als Sprengmittel (E) wenigstens ein Sprengmittel ausgewählt aus der Gruppe umfassend vernetzte Natriumcarboxymethylcellulose (Crosscamellose), modifizierte Maisstärke, Natriumcarboxymethylstärke, vernetztes Polyvinylpyrrolidon (Crosspovidon) vorliegt.

9. Darreichungsform nach einem der vorstehenden Ansprüche, **dadurch gekennzeichnet, dass** als weitere Hilfsstoffe (B) Formulierungshilfsstoffe (B1) vorliegen.

10. Darreichungsform nach Anspruch 9, **dadurch gekennzeichnet, dass** als Formulierungshilfsstoffe (B1) wenigstens eine vorzugsweise temperaturstabile Verbindung ausgewählt aus der Gruppe umfassend Weichmacher, Antioxidantien, Redoxstabilisatoren und Füllstoffe vorliegen.

11. Darreichungsform nach einem der vorstehenden Ansprüche, **dadurch gekennzeichnet, dass** als Zusatzhilfsstoff (B2) ein Füllstoff ausgewählt aus der Gruppe bestehend aus mikrokristalliner Cellulose, Calciumdihydrogenphosphat, Zucker wie Lactose, Pulvercellulose und/oder Polyvinylpyrrolidon vorliegt.

12. Verfahren zur Herstellung einer Darreichungsform gemäß einem der Ansprüche 1 bis 11 umfassend die folgenden Verfahrensschritte:
(i)
a. Mischen der Komponenten (A), (C), ggf. (B1), ggf. (D) und ggf. zumindest einen Teil des Sprengmittels (E);
b. ggf. Vorformen der aus Schritt a) erhaltenen Mischung, vorzugsweise unter Wärme und/oder Kraft-Einwirkung auf die aus a) erhaltenen Mischung ohne Erweichung der Komponente (C);
c. Härten der Mischung unter Wärme-/und Kraft-Einwirkung, wobei die Wärme während und/oder vor der Krafteinwirkung zugeführt wird und dazu ausreicht, die Komponente (C) wenigstens bis zu ihrem Erweichungspunkt zu erwärmen;
d. Aufteilung der gehärteten Mischung in Teilmassen;
e. Vereinzeln der gehärteten Teilmassen und ggf. Formeln;
f. Mischen mit dem aus a) verbliebenen Teil des Sprengmittels (E) und weiteren Hilfsstoffen (B2);
g. und ggf. Endformen oder Abfüllen zu einer Dosiseinheit der Darreichungsform;
oder
(ii)
a. Mischen der Komponenten (A), (C), ggf. (B1), ggf. (D) und ggf. zumindest eines Teils des Sprengmittels (E) unter Zugabe eines Lösungsmittels für das Polymer (C) zumindest in solchen Mengen, dass die Mischung gleichzeitig befeuchtet und formbar wird,
b. Aufteilung der formbaren Masse vor oder nach Trocknung in Teilmassen,
c. Vereinzeln der ggf. nach einer weiteren Verteilung erhaltenen Teilmassen und ggf. Formen,
d. Mischen mit dem aus a) verbliebenen Teil des Sprengmittels (E) und weiteren Hilfsstoffen (B2) und
e. ggf. Endformen und/oder Abfüllen zu einer Dosiseinheit der Darreichungsform.

## Claims

1. Multiparticulate dosage form with impeded abuse potential comprising
- at least one active ingredient with abuse potential (A), which has a psychotropic effect, selected from the group of the opioids;
- at least one synthetic or natural polymer (C);
- optionally at least one natural, semi-synthetic or synthetic wax (D);
- at least one disintegrant (E) which is at least partially mixed with the particles of the dosage form;
- an excipient (B2), which is not a constituent of the particle, selected from the group of fillers; and
- optionally one or more further physiologically compatible auxiliaries (B),
wherein the individual particles of the dosage form have a fracture resistance of at least 500 N measured according to Pharm. Eur. and an active ingredient release of at least 75% after 45 minutes measured according to Pharm. Eur. in the paddle stirrer apparatus with sinker in 600 ml of aqueous buffer solution with a pH of 1.2 at 37°C and 75 revolutions per minute.

2. Dosage form according to Claim 1, **characterized in that** it is in the form of microtablets, micropellets, granules, spheroids, beads or pellets.

3. Dosage form according to either of Claims 1 or 2, **characterized in that** the polymer (C) is at least one polymer selected from the group comprising polyalkylene oxides, polyethylenes, polypropylenes, polyvinyl chloride, polycarbonates, polystyrenes, polyacrylates and copolymers thereof.

4. Dosage form according to any of the preceding claims, **characterized in that** the polymer (C) is at least one polyalkylene oxide selected from the group comprising polymethylene oxide, polyethylene oxide, polypropylene oxide, copolymers thereof, block copolymers thereof and mixtures thereof, preferably at least one polyethylene oxide.

5. Dosage form according to Claim 3 or 4, **characterized in that** the polyalkylene oxide has a molecular weight of at least 0.5 x 10⁶ based on rheological measurements.

6. Dosage form according to Claim 5, **characterized in that** the polyalkylene oxide has a molecular weight of at least 1 x 10⁶ based on rheological measurements.

7. Dosage form according to any of the preceding claims, **characterized in that** it has 0.1 to 15% by weight, preferably 1 to 10% by weight, particularly preferably 3 to 7% by weight of at least one disintegrant (E), based on the total weight of the dosage form.

8. Dosage form according to any of the preceding claims, **characterized in that** the disintegrant (E) is at least one disintegrant selected from the group comprising crosslinked sodium carboxymethylcellulose (croscarmellose), modified maize starch, sodium carboxymethyl starch, crosslinked polyvinylpyrrolidone (crospovidone).

9. Dosage form according to any of the preceding claims, **characterized in that** the further auxiliary (B) is formulation auxiliary (B1).

10. Dosage form according to Claim 9, **characterized in that** the formulation auxiliary (B1) is at least one preferably temperature-stable compound selected from the group comprising plasticizers, antioxidants, redox stabilizers and fillers.

11. Dosage form according to any of the preceding claims, **characterized in that** the excipient (B2) is a filler selected from the group consisting of microcrystalline cellulose, calcium dihydrogen phosphate, sugars such as lactose, cellulose powder and/or polyvinylpyrrolidone.

12. Method for preparing a dosage form according to any of Claims 1 to 11, comprising the following method steps:
(i)
a. mixing components (A), (C), optionally (B1), optionally (D) and optionally at least a portion of the disintegrant (E);
b. optionally preforming the mixture obtained from step a), preferably by the effect of heat and/or force on the mixture obtained from a) without softening component C);
c. hardening the mixture by the effect of heat and force, wherein the heat is supplied during and/or before the effect of force and which is thereby sufficient to heat component (C) at least up to its softening point;
d. dividing the hardened mixture into partial masses;
e. separating the hardened partial masses and optionally forms;
f. mixing with the portion of the disintegrant (E) remaining from a) and further excipients (B2);
g. and optionally final shaping or filling of a dosing unit of the dosage form;
or
(ii)
a. mixing components (A), (C), optionally (B1), optionally (D) and optionally at least a portion of the disintegrant (E) with addition of a solvent for the polymer (C) at least in amounts such that the mixture is at the same time wetted and formable,
b. dividing the formable mass before or after drying in partial masses,
c. separating the optionally partial masses and optionally forms obtained after a further division,
d. mixing with the portion of the disintegrant (E) remaining from a) and further excipients (B2) and
e. optionally final shaping and/or filling of a dosing unit of the dosage form.

## Revendications

1. Forme pharmaceutique multiparticulaire dont l'abus est rendu plus difficile, comprenant :
- au moins un agent actif à potentiel d'accoutumance (A), qui présente un effet psychotrope, choisi dans le groupe des opioïdes ;
- au moins un polymère synthétique ou naturel (C) ;
- éventuellement au moins une cire naturelle, semi-synthétique ou synthétique (D) ;
- au moins un agent de désintégration (E), qui est au moins partiellement mélangé avec les particules de la forme médicamenteuse ;
- un adjuvant additif (B2), qui ne fait pas partie des particules, choisi dans le groupe des charges ; et
- éventuellement un ou plusieurs adjuvants physiologiquement compatibles supplémentaires (B),
les particules individuelles de la forme médicamenteuse présentant une résistance à la rupture d'au moins 500 N, mesurée selon Pharm. Eur. et une libération d'agent actif d'au moins 75 % après 45 minutes mesurée selon Pharm. Eur. dans l'appareil d'agitation à pales avec un poids dans 600 ml d'une solution tampon aqueuse ayant un pH de 1,2 à 37 °C et 75 tours par minute.

2. Forme médicamenteuse selon la revendication 1, **caractérisée en ce qu'**elle se présente sous la forme de microcomprimés, de micropastilles, de granulats, de sphéroïdes, de perles ou de pastilles.

3. Forme médicamenteuse selon l'une quelconque des revendications 1 ou 2, **caractérisée en ce qu'**en tant que polymère (C), au moins un polymère choisi dans le groupe comprenant les polyoxydes d'alkylène, les polyéthylènes, les polypropylènes, les polychlorures de vinyle, les polycarbonates, les polystyrènes, les polyacrylates et leurs copolymères est présent.

4. Forme médicamenteuse selon l'une quelconque des revendications précédentes, **caractérisée en ce qu'**en tant que polymère (C), au moins un polyoxyde d'alkylène choisi dans le groupe comprenant les polyoxydes de méthylène, les polyoxydes d'éthylène, les polyoxydes de propylène, leurs copolymères, leurs copolymères séquencés et leurs mélanges, de préférence au moins un polyoxyde d'éthylène, est présent.

5. Forme médicamenteuse selon la revendication 3 ou 4, **caractérisée en ce que** le polyoxyde d'alkylène présente un poids moléculaire d'au moins 0,5 x 10⁶, d'après des mesures rhéologiques.

6. Forme médicamenteuse selon la revendication 5, **caractérisée en ce que** le polyoxyde d'alkylène présente un poids moléculaire d'au moins 1 x 10⁶, d'après des mesures rhéologiques.

7. Forme médicamenteuse selon l'une quelconque des revendications précédentes, **caractérisée en ce qu'**elle comprend 0,1 à 15 % en poids, de préférence 1 à 10 % en poids, de manière particulièrement préférée 3 à 7 % en poids, par rapport au poids total de la forme médicamenteuse, d'au moins un agent de désintégration (E).

8. Forme médicamenteuse selon l'une quelconque des revendications précédentes, **caractérisée en ce qu'**en tant qu'agent de désintégration, au moins un agent de désintégration choisi dans le groupe comprenant la carboxyméthylcellulose de sodium réticulée (crosscamellose), l'amidon de maïs modifié, le carboxyméthyl-amidon de sodium, la polyvinylpyrrolidone réticulée (crosspovidone), est présent.

9. Forme médicamenteuse selon l'une quelconque des revendications précédentes, **caractérisée en ce qu'**en tant qu'adjuvants supplémentaires (B), des adjuvants de formulation (B1) sont présents.

10. Forme médicamenteuse selon la revendication 9, **caractérisée en ce qu'**en tant qu'adjuvants de formulation (B1), au moins un composé de préférence stable en température, choisi dans le groupe comprenant les plastifiants, les antioxydants, les stabilisateurs redox et les charges, est présent.

11. Forme médicamenteuse selon l'une quelconque des revendications précédentes, **caractérisée en ce qu'**en tant qu'adjuvant additif (B2), une charge choisie dans le groupe constitué par la cellulose microcristalline, le dihydrogénophosphate de calcium, les sucres tels que le lactose, la cellulose en poudre et/ou la polyvinylpyrrolidone, est présente.

12. Procédé de fabrication d'une forme médicamenteuse selon l'une quelconque des revendications 1 à 11, comprenant les étapes de procédé suivantes :
(i)
a. le mélange des composants (A), (C), éventuellement (B1), éventuellement (D) et éventuellement au moins une partie de l'agent de désintégration (E) ;
b. éventuellement le pré-façonnage du mélange obtenu à l'étape a), de préférence sous l'effet de chaleur et/ou d'une force sur le mélange obtenu en a) sans ramollissement du composant (C) ;
c. le durcissement du mélange sous l'effet de chaleur et/ou d'une force, la chaleur étant introduite pendant et/ou avant l'effet de la force et étant suffisante pour chauffer le composant (C) au moins jusqu'à son point de ramollissement ;
d. la division du mélange durci en masses partielles ;
e. la séparation des masses partielles durcies et éventuellement le façonnage ;
f. le mélange avec la partie de l'agent de désintégration (E) restant de a) et des adjuvants supplémentaires (B2) ;
g. et éventuellement le façonnage final ou le remplissage pour obtenir une unité de dose de la forme médicamenteuse ;
ou
(ii)
a. le mélange des composants (A), (C), éventuellement (B1), éventuellement (D) et éventuellement au moins une partie de l'agent de désintégration (E) avec ajout d'un solvant pour le polymère (C) au moins en quantités telles que le mélange soit simultanément humidifié et façonnable,
b. la division de la masse façonnable avant ou après le séchage en masses partielles,
c. la séparation des masses partielles obtenues éventuellement après une division supplémentaire et éventuellement le façonnage,
d. le mélange avec la partie de l'agent de désintégration (E) restant de a) et des adjuvants supplémentaires (B2), et
e. éventuellement le façonnage final et/ou le remplissage pour obtenir une unité de dose de la forme médicamenteuse.
